# EUROPEAN PATENT SPECIFICATION

(11) **EP 3 297 613 B1**
(45) Date of publication and mention of the grant of the patent: **03.09.2025**
(21) Application number: 16712656.4
(22) Date of filing: 17.03.2016
(51) Int. Cl.: A61K 9/51, A61K 31/436, A61K 31/704, A61P 13/12, A61K 49/00

(54) **MESOSCALE NANOPARTICLES FOR SELECTIVE TARGETING TO THE KIDNEY AND METHODS OF THEIR THERAPEUTIC USE**
MITTELGROSSE NANOPARTIKEL ZUR SELEKTIVEN APPLIKATION AN DER NIERE UND DEREN MEDIZINISCHE VERWENDUNG
NANOPARTICULES MOYENNES POUR L'APPLICATION SELECTIVE AU REIN ET L'UTILISATION MEDICALE

(30) Priority: 20.03.2015 US 201562136104 P
(43) Date of publication of application: 28.03.2018
(73) Proprietor: Research Foundation of the City University of New York, New York, NY 10036 (US)
(72) Inventor: HELLER, Daniel A., New York, New York 10065 (US); WILLIAMS, Ryan M., New York, New York 10028 (US)
(74) Representative: Barker Brettell LLP
(86) International application number: PCT/US2016/022879
(87) International publication number: WO 2016/153920

(56) References cited:
- WO-A2-2007/001448
- US-A1- 2006 127 386
- US-A1- 2008 166 414
- LI Y-P ET AL: "PEGylated PLGA nanoparticles as protein carriers: synthesis, preparation and biodistribution in rats", JOURNAL OF CONTROLLED RELEASE, ELSEVIER, AMSTERDAM, NL, vol. 71, no. 2, 2 April 2001 (2001-04-02), pages 203 - 211, XP004232020, ISSN: 0168-3659, DOI: 10.1016/S0168-3659(01)00218-8
- DINESH SHENOY ET AL: "Poly(Ethylene Oxide)-Modified Poly([beta]-Amino Ester) Nanoparticles as a pH-Sensitive System for Tumor-Targeted Delivery of Hydrophobic Drugs: Part 2. In Vivo Distribution and Tumor Localization Studies", PHARMACEUTICAL RESEARCH, KLUWER ACADEMIC PUBLISHERS-PLENUM PUBLISHERS, NL, vol. 22, no. 12, 1 December 2005 (2005-12-01), pages 2107 - 2114, XP019370768, ISSN: 1573-904X, DOI: 10.1007/S11095-005-8343-0
- JINBIN LIU ET AL: "Passive Tumor Targeting of Renal-Clearable Luminescent Gold Nanoparticles: Long Tumor Retention and Fast Normal Tissue Clearance", JOURNAL OF THE AMERICAN CHEMICAL SOCIETY, vol. 135, no. 13, 3 April 2013 (2013-04-03), US, pages 4978 - 4981, XP055268262, ISSN: 0002-7863, DOI: 10.1021/ja401612x
- KUMAR A ET AL: "Multifunctional magnetic nanoparticles for targeted delivery", NANOMEDICINE: NANOTECHNOLOGY, BIOLOGY AND MEDICINE, ELSEVIER, NL, vol. 6, no. 1, 1 February 2010 (2010-02-01), pages 64 - 69, XP027501935, ISSN: 1549-9634, [retrieved on 20100201], DOI: 10.1016/J.NANO.2009.04.002
- KRISTI L. HULTMAN ET AL: "Magnetic Resonance Imaging of Major Histocompatibility Class II Expression in the Renal Medulla Using Immunotargeted Superparamagnetic Iron Oxide Nanoparticles", ACS NANO, vol. 2, no. 3, 1 March 2008 (2008-03-01), US, pages 477 - 484, XP055268269, ISSN: 1936-0851, DOI: 10.1021/nn700400h
- ADAM J COLE ET AL: "Magnetic brain tumor targeting and biodistribution of long-circulating PEG-modified, cross-linked starch-coated iron oxide nanoparticles", BIOMATERIALS, ELSEVIER SCIENCE PUBLISHERS BV., BARKING, GB, vol. 32, no. 26, 5 May 2011 (2011-05-05), pages 6291 - 6301, XP028097285, ISSN: 0142-9612, [retrieved on 20110513], DOI: 10.1016/J.BIOMATERIALS.2011.05.024
- ILLUM L ET AL: "The organ distribution and circulation time of intravenously injected colloidal carriers sterically stabilized with a blockcopolymer - poloxamine 908", LIFE SCIENCES, PERGAMON PRESS, OXFORD, GB, vol. 40, no. 4, 26 January 1987 (1987-01-26), pages 367 - 374, XP023767505, ISSN: 0024-3205, [retrieved on 19870126], DOI: 10.1016/0024-3205(87)90138-X
- DOLMAN M E M ET AL: "Drug targeting to the kidney: Advances in the active targeting of therapeutics to proximal tubular cells", ADVANCED DRUG DELIVERY REVIEWS, ELSEVIER, AMSTERDAM, NL, vol. 62, no. 14, 30 November 2010 (2010-11-30), pages 1344 - 1357, XP027511625, ISSN: 0169-409X, [retrieved on 20100816], DOI: 10.1016/J.ADDR.2010.07.011

## Description

### Cross Reference to Related Application

This application claims the benefit of U.S. Application Serial No. 62/136,104 filed on March 20, 2015.

### Field of the Invention

This invention relates generally to nanoparticles and methods of their manufacture and therapeutic use. In particular embodiments, the invention relates to mesoscale nanoparticles for selective targeting of the renal proximal tubule epithelium.

### Government Funding

This invention was made with government support under Grant No. DP2-HD075698 awarded by National Institutes of Health. The government has certain rights in this invention.

### Background

Specific physiological parameters that enhance delivery to diseases sites, including the enhanced permeability and retention (EPR) effect to localize nanoparticles in tumors, have been exploited to target specific sites in tissues. However, the EPR effect has not yet been shown to result in significant targeting in human patients, possibly due to low accumulation in small tumors and disseminated disease. Recently, "active" targeting of disease sites have been investigated via functionalization of nanoparticles with molecular recognition moieties such as antibodies, small molecules, or aptamers. This general approach has generated positive pre-clinical results, some of which have progressed to clinical trials.

Often, and sometimes irrespective of a molecular targeting element, nanoparticles may localize in one of several organs due to the particle surface chemistry, size, and zeta potential. The purposeful application of this mechanism may allow for the treatment of diseases regardless of the expression of molecular targets or the size of a lesion. The delivery of targeted agents to specific organs and tissues may obviate off-target effects in systemic delivery such as neutropenia or GI toxicity. To employ this targeting approach, there is a need to understand the properties of nanoparticles that cause differential biodistribution in specific organs and cell types.

Several kidney diseases may benefit from the development of nanoparticle therapeutics which allow for site-directed accumulation, controlled temporal release, and protection of a therapeutic payload. Among candidate diseases are lupus, glomerulonephritis, and renal cell carcinoma (RCC), which often arises in the proximal tubules. Pharmacological therapeutic options for these diseases are limited, thus there is a strong need to increase the efficacy and decrease side effects of current drugs.

Only a few instances of previous work have attempted to specifically target the kidney using nanoparticles. First, one technology was limited to kidney targeting where accumulation in the kidney is only marginally greater than the next highest organ and experiences rapid degradation *in vivo* (Fischer et al. PLOS ONE 2014 9: e93342 and Shenoy et al. Pharmaceutical Research 2005 22: 2107-2114). A second technology synthesized nanoparticles that can accumulate in the glomerulus of kidneys (Choi et al. PNAS 2011 108: 6656-6661). However, these nanoparticles exhibit significantly greater accumulation in the liver and spleen compared to the kidney. While other examples of nanoparticle technologies synthesized with PLGA-PEG exist, none accumulates in the kidneys. Moreover, although low-molecular weight polymers, peptides, and proteins have been shown accumulate in kidney proximal tubules, these platforms are quickly cleared from the body and do not carry a large therapeutic payload (Dolman et al Advanced Drug Delivery Reviews 2010 62:1344-1357). Li et al (2001) describes PEGylated poly(lactic-co-glycolic acid) nanoparticles as protein and peptide carriers. Dinesh Shenoy et al (2005) describes poly(ethylene oxide)-modified poly([beta]-amino ester) nanoparticles as a pH-sensitive system for tumor-targeted delivery of hydrophobic drugs.

Therefore, there is a strong need for nanoparticles that specifically target and accumulate in the kidney, exhibit high stability over a long period of time, and provide the ability to carry and deliver large therapeutic payloads to the kidney.

### Summary of invention

Described herein are mesoscale nanoparticles for selective targeting to the kidney. A drug carrier nanoparticle has been synthesized that can specifically target the proximal tubules of the kidneys. The nanoparticles accumulate in the kidneys to a greater extent than other organs (e.g., up to 3 or more times greater in the kidney than any other organ). They can encapsulate many classes of drug molecules. The nanoparticles are biodegradable and release the drug as they degrade. The particles can sustainably release a drug within the kidneys for up to two months. The nanoparticles are useful for the treatment of diseases that affect the proximal tubules, such as heart failure, liver cirrhosis, hypertension, and renal failure; the study of relative blood flow to the renal cortex and medulla; and delivery of agents to treat gout.

Because of the kidney-targeting ability of the drug carrier nanoparticles, treatment of the kidney can now be performed using therapeutic agents that are currently not used for kidney treatment and/or that are currently experimental. Various therapeutic agents (e.g., TGFb inhibitors, mTOR inhibitors, everolimus, kinase inhibitors) have not been usable for kidney disease treatment due to poor pharmacokinetics. By the time the kidneys respond to a delivered therapeutic agent, toxicity side-effects occur elsewhere in the body. Accordingly, the mesoscale nanoparticles disclosed herein can selectively deliver therapeutic agents (e.g., therapeutic agents that are typically not used for kidney disease treatment due to poor pharmacokinetics) to the kidney while minimizing toxicity elsewhere in the body.

In one aspect, the invention is directed to a mesoscale nanoparticle composition as defined in claim 1.

In certain embodiments, a base of PLGA is modified. In certain embodiments, the PLGA has a molecular weight from 7 kDa to 54 kDa. In certain embodiments, the polyethylene glycol has a molecular weight from 2 kDa to 10 kDa (e.g., from 4 kDa to 7 kDa, e.g., 5 kDa). In certain embodiments, the surface coating is selected from the group consisting of polyethylene glycol (PEG), PEG-carboxylic acid, PEG-carboxylic acid-DMAB, and methoxy PEG. In certain embodiments, the weight ratio of PEG to PLGA is from 9% to 13% (e.g., prior to synthesis of the mesoscale nanoparticle composition).

In certain embodiments, the targeted chemotherapeutic is doxorubicin, sorafenib, or sunitinib; the metabolic targeting therapeutic is STF-31, CPI-613, or Fasentin; the TGFbeta inhibitor is a kinase inhibitors (e.g., LY2157299 (galunisertib), SD-208, SB505124); the Nf kappa B inhibitor is Pyrrolidine dithiocarbamate, quinazoline, BMS-345541, or BAY-11 -7085.

In certain embodiments, the therapeutic agent is noncovalently associated with the nanoparticle, e.g., encapsulated within the surface coating. In certain embodiments, the therapeutic agent is attached to the nanoparticle (e.g., non-covalently attached or covalently bound to a moiety of the surface coating).

In certain embodiments, the mesoscale nanoparticle composition comprises a radiolabel noncovalently associated with the nanoparticle and/or attached to the nanoparticle.

In another aspect, the invention is directed to a mesoscale nanoparticle composition for use as defined in claim 10.

In certain embodiments, the disease or condition is a member selected from the group consisting of renal carcinoma, acute kidney disease, chronic kidney disease, heart failure, liver cirrhosis, hypertension, and renal failure.

In another aspect, the invention is directed to a method for monitoring a patient as defined in claim 11.

In certain embodiments, the administered mesoscale nanoparticle composition demonstrates selective targeting of kidneys of the patient such that concentration of the nanoparticle in the kidneys is at least 1.5 times greater than concentration of the nanoparticle in any of the heart, lung, liver, or spleen of the patient (e.g., at least 1.5 times greater, at least 2 times greater, at least 3 times greater, 4 times greater, at least 5 times greater, at least 6 times greater, or at least 7 times greater) at a given time following administration of the composition, wherein the given time is from 3 days to 2 months following administration.

Elements of embodiments involving one aspect of the invention (e.g., methods) can be applied in embodiments involving other aspects of the invention (e.g., systems), and vice versa. The invention is defined by the claims and any other aspect or embodiment set forth herein not falling within the scope of the claims is for information only.

### Definitions

In order for the present disclosure to be more readily understood, certain terms are first defined below. Additional definitions for the following terms and other terms are set forth throughout the specification.

In this application, the use of "or" means "and/or" unless stated otherwise. As used in this application, the term "comprise" and variations of the term, such as "comprising" and "comprises," are not intended to exclude other additives, components, integers or steps. As used in this application, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

***"Administration"***: The term "administration" refers to introducing a substance into a subject. In general, any route of administration may be utilized including, for example, parenteral (e.g., intravenous), oral, topical, subcutaneous, peritoneal, intraarterial, inhalation, vaginal, rectal, nasal, introduction into the cerebrospinal fluid, or instillation into body compartments. In some embodiments, administration is oral. Additionally or alternatively, in some embodiments, administration is parenteral. In some embodiments, administration is intravenous.

***"Associated"*:** As used herein, the term "associated" typically refers to two or more entities in physical proximity with one another, either directly or indirectly (e.g., via one or more additional entities that serve as a linking agent), to form a structure that is sufficiently stable so that the entities remain in physical proximity under relevant conditions, *e*.*g*., physiological conditions. In some embodiments, associated moieties are covalently linked to one another. In some embodiments, associated entities are non-covalently linked. In some embodiments, associated entities are linked to one another by specific non-covalent interactions (e.g., by interactions between interacting ligands that discriminate between their interaction partner and other entities present in the context of use, such as, for example. streptavidin/avidin interactions, antibody/antigen interactions, *etc*.). Alternatively or additionally, a sufficient number of weaker non-covalent interactions can provide sufficient stability for moieties to remain associated. Exemplary non-covalent interactions include, but are not limited to, electrostatic interactions, hydrogen bonding, affinity, metal coordination, physical adsorption, host-guest interactions, hydrophobic interactions, pi stacking interactions, van der Waals interactions, magnetic interactions, electrostatic interactions, dipole-dipole interactions, *etc.*

***"Biocompatible"*:** The term "biocompatible", as used herein is intended to describe materials that do not elicit a substantial detrimental response *in vivo.* In certain embodiments, the materials are "biocompatible" if they are not toxic to cells. In certain embodiments, materials are "biocompatible" if their addition to cells *in vitro* results in less than or equal to 20% cell death, and/or their administration *in vivo* does not induce inflammation or other such adverse effects. In certain embodiments, materials are biodegradable.

***"Biodegradable"***: As used herein, "biodegradable" materials are those that, when introduced into cells, are broken down by cellular machinery (*e*.*g*., enzymatic degradation) or by hydrolysis into components that cells can either reuse or dispose of without significant toxic effects on the cells. In certain embodiments, components generated by breakdown of a biodegradable material do not induce inflammation and/or other adverse effects *in vivo.* In some embodiments, biodegradable materials are enzymatically broken down. Alternatively or additionally, in some embodiments, biodegradable materials are broken down by hydrolysis. In some embodiments, biodegradable polymeric materials break down into their component polymers. In some embodiments, breakdown of biodegradable materials (including, for example, biodegradable polymeric materials) includes hydrolysis of ester bonds. In some embodiments, breakdown of materials (including, for example, biodegradable polymeric materials) includes cleavage of urethane linkages.

***"Carrier"***: As used herein, "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

***"Substantially"***: As used herein, the term "substantially", and grammatic equivalents, refer to the qualitative condition of exhibiting total or near-total extent or degree of a characteristic or property of interest. One of ordinary skill in the art will understand that biological and chemical phenomena rarely, if ever, go to completion and/or proceed to completeness or achieve or avoid an absolute result.

***"Subject"*:** As used herein, the term "subject" includes humans and mammals (e.g., mice, rats, pigs, cats, dogs, and horses). In many embodiments, subjects are be mammals, particularly primates, especially humans. In some embodiments, subjects are livestock such as cattle, sheep, goats, cows, swine, and the like; poultry such as chickens, ducks, geese, turkeys, and the like; and domesticated animals particularly pets such as dogs and cats. In some embodiments (e.g., particularly in research contexts) subject mammals will be, for example, rodents (e.g., mice, rats, hamsters), rabbits, primates, or swine such as inbred pigs and the like.

***"Therapeutic agent"***: As used herein, the phrase "therapeutic agent" refers to any agent that has a therapeutic effect and/or elicits a desired biological and/or pharmacological effect, when administered to a subject.

***"Treatment"*:** As used herein, the term "treatment" (also "treat" or "treating") refers to any administration of a substance that partially or completely alleviates, ameliorates, relives, inhibits, delays onset of, reduces severity of, and/or reduces incidence of one or more symptoms, features, and/or causes of a particular disease, disorder, and/or condition. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

Drawings are presented herein for illustration purposes, not for limitation.

### Brief description of drawings

The foregoing and other objects, aspects, features, and advantages of the present disclosure will become more apparent and better understood by referring to the following description taken in conduction with the accompanying drawings, in which:
FIG. 1 shows the primary organ of localization for non-targeted nanoparticles administered intravenously to healthy mice was plotted according to the particle diameter and degree of surface passivation. Numbers correlate with literature references listed in Table 2 while the - symbol denotes A-MNPs, + symbol denotes C-MNPs particles, ★denotes N-MNPs, and 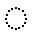 denotes O-MNPs synthesized in this study. Black references denote liver localization, blue denotes spleen, red denotes the kidneys, and orange denotes other (stomach or lymph nodes).

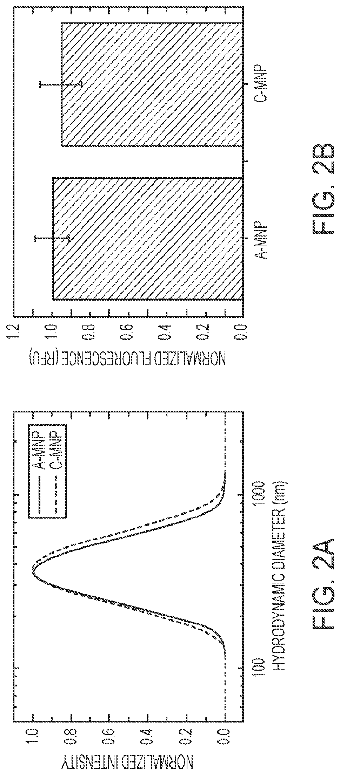

FIG. 2A shows normalized intensity distribution of nanoparticle diameter as measured by dynamic light scattering (DLS).
FIG. 2B shows normalized fluorescence from an equal weight of each nanoparticle formulation.
FIGS. 3A - 3E show MNP characterization.

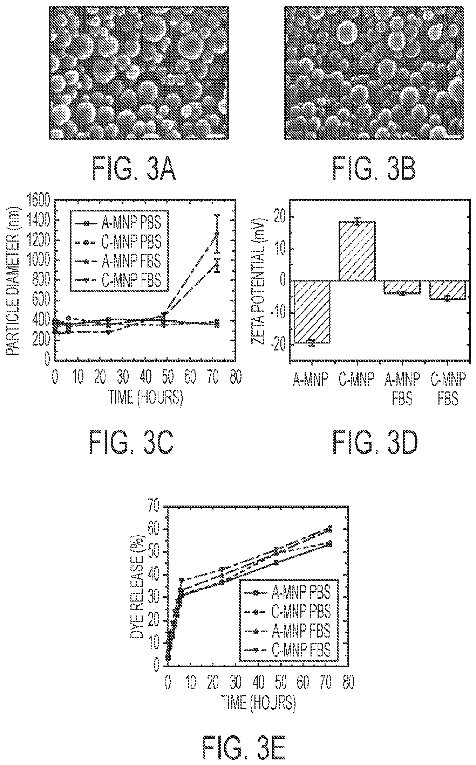

FIGS. 3A and 3B show scanning electron micrographs of A-MNPs and C-MNPs, respectively. Scale bars are 300 nm for both images.
FIG. 3C shows dynamic nanoparticle stability measurement by dynamic light scattering (DLS) in phosphate-buffered saline (PBS) and fetal bovine serum (FBS).
FIG. 3D shows nanoparticle ζ potentials in water and FBS.
FIG. 3E shows nanoparticle dye release assay in PBS and FBS.

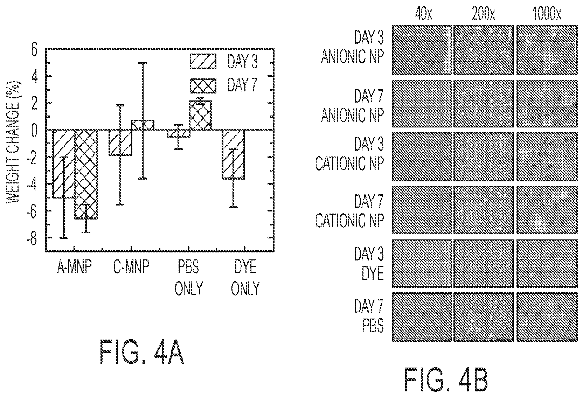

FIGS. 4A and 4B show mesoscale nanoparticle (MNP) toxicity studies.
FIG. 4A shows percent change in weight from injection to day 3 or day 7 for mice with the given treatment (Mean ± SD) (*=p<0.05).
FIG. 4B shows H&E stained renal tissue from mice with the noted treated on the given day. Scale bar for 40x column: 10 µm; 200x column: 100 µm; 1000x column: 100 µm.

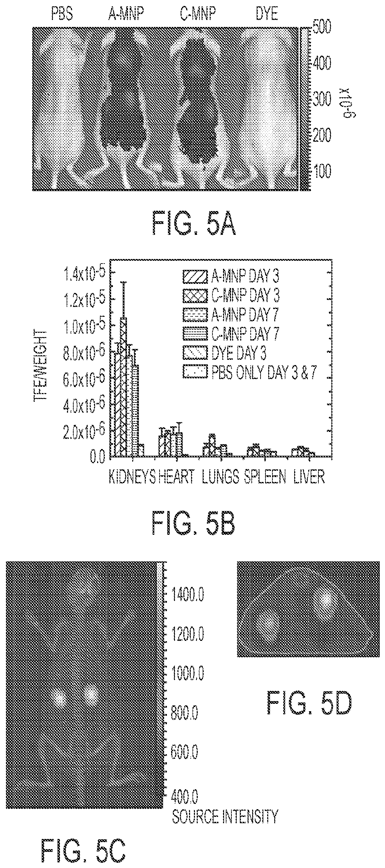

FIGS. 5A-5D show *in vivo* biodistribution of MNPs.
FIG. 5A shows dorsal image of mice treated with: PBS, 50 mg/kg A-MNPs, 50 mg/kg C-MNPs, and an equal molar weight of free dye on the day they were sacrificed.
FIG. 5B shows *ex vivo* organ fluorescence from mice injected with MNPs, dye, or PBS normalized by total organ weight (Mean ± SD).
FIG. 5C shows fluorescence plus CT overlay focused on the kidneys of a mouse treated with A-MNPs showing localization and relatively homogenous distribution throughout the kidneys.
FIG. 5D shows fluorescence plus CT transaxial section of a mouse treated with A-MNPs showing bright fluorescence throughout the kidneys.

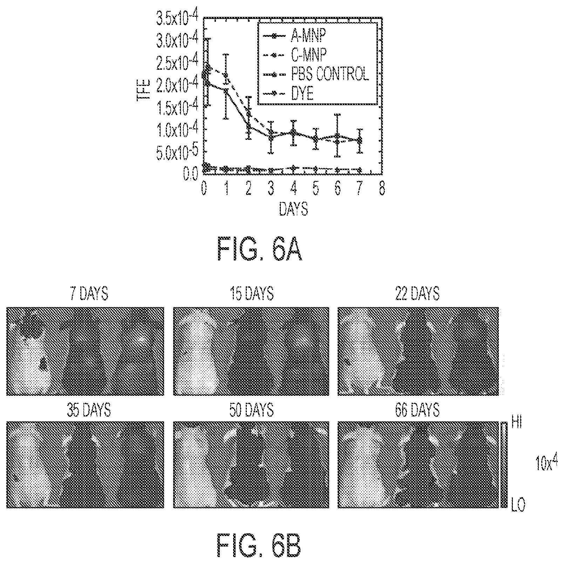

FIGS. 6A and 6B show MNP fate *in vivo.*
FIG. 6A shows nanoparticle fluorescence measured from the kidney region of live mice measured daily for 7 days after injection (Mean ± SD).
FIG. 6B shows nanoparticle emission from mice treated with (L to R): PBS, A-MNP, and C-MNP, measured up to 66 days after injection.

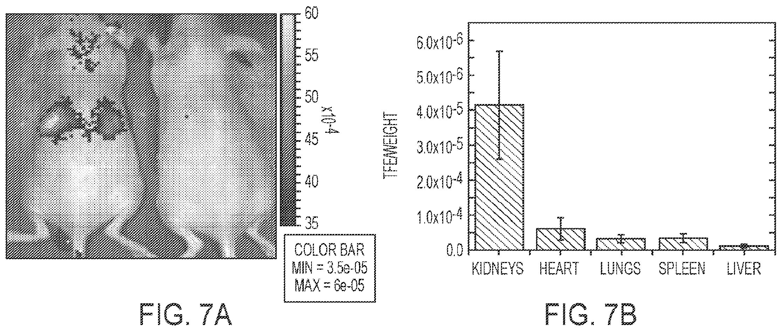

FIGS. 7A and 7B show distribution of neutral mesoscale nanoparticles (N-MNPs) injected into mice.
FIG. 7A shows *in vivo* fluorescence image 30 minutes following injection. The mouse on the left was treated with 50 mg/kg N-MNPs. The mouse on the right was treated with PBS.
FIG. 7B shows that *ex vivo* fluorescence of each organ was normalized by dividing total fluorescence by organ weight (Mean ± SD). p<0.05 for kidneys versus each organ.

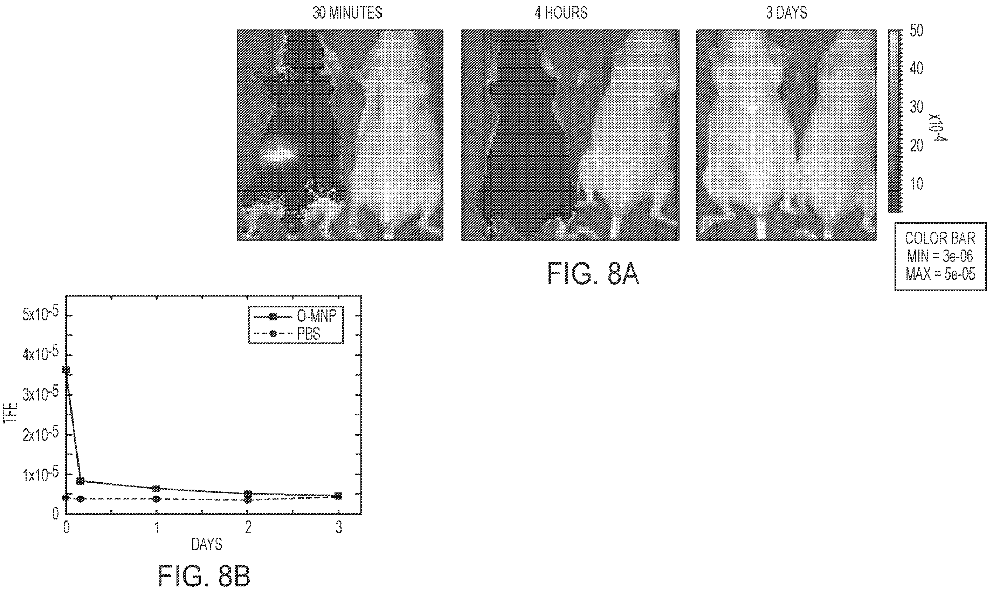

FIGS. 8A and 8B show localization of opsonizing PLGA nanoparticles without PEG (O-MNPs).
FIG. 8A shows *in vivo* fluorescence images of mice imaged ventrally at 30 minutes, 4 hours, and 3 days post-injection of O-MNPs (left) and PBS (right).
FIG. 8B shows quantification of *in vivo* liver fluorescence (Mean ± SD for treated mice).

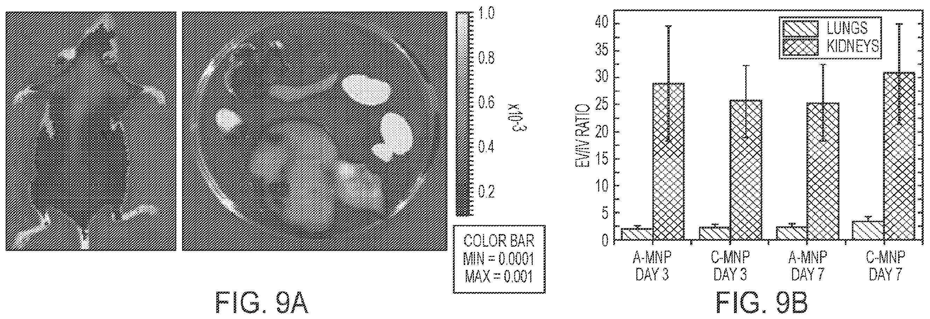

FIGS. 9A and 9B show comparison of *ex vivo* to *in vivo* fluorescence.
FIG. 9A shows emission from mouse carcass after removal of major organs (left panel) andemission from organs removed from the mouse (right panel). From top center, clockwise: spleen, left kidney, right kidney, liver, heart, lungs.
FIG. 9B shows *ex vivo* to *in vivo* ratio of fluorescence from lungs or kidneys of mice imaged at day 3 or 7 with A-MNPs or C-MNPs (Mean ± SD).

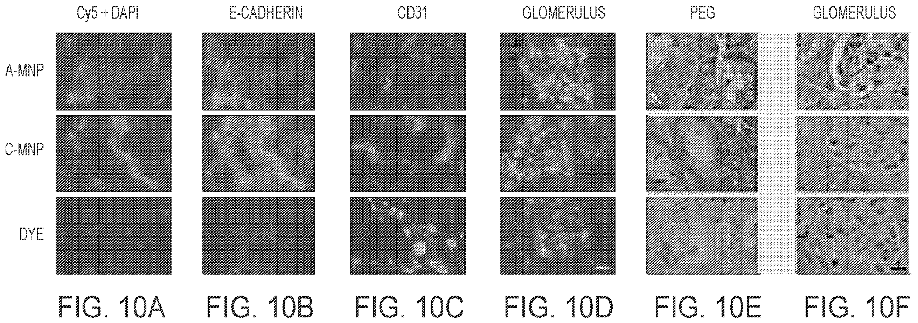

FIGS. 10A - 10H show tissue-level localization of MNPs. Representative micrographs of renal tissue from mice at day 3 after nanoparticle administration.
FIGS. 10A - 10D show immunofluorescence images with blue denoting DAPI stain for cell nuclei and red denoting fluorescence from nanoparticles (e.g., excluding bright red blood cells which show standard autofluorescence).
FIG. 10A shows fluorescence from nanoparticles and nuclei alone.
FIG. 10B shows the same field as FIG. 10A, with green denoting E-cadherin. Green denotes CD31 in FIGS. 10C and 10D.
FIGS. 10E - 10F show immunohistochemistry with anti-PEG antibody showing particle localization (brown).
FIGS. 10A - 10C and 10E are kidney tubules. FIGS. 10D and 10F are glomeruli.

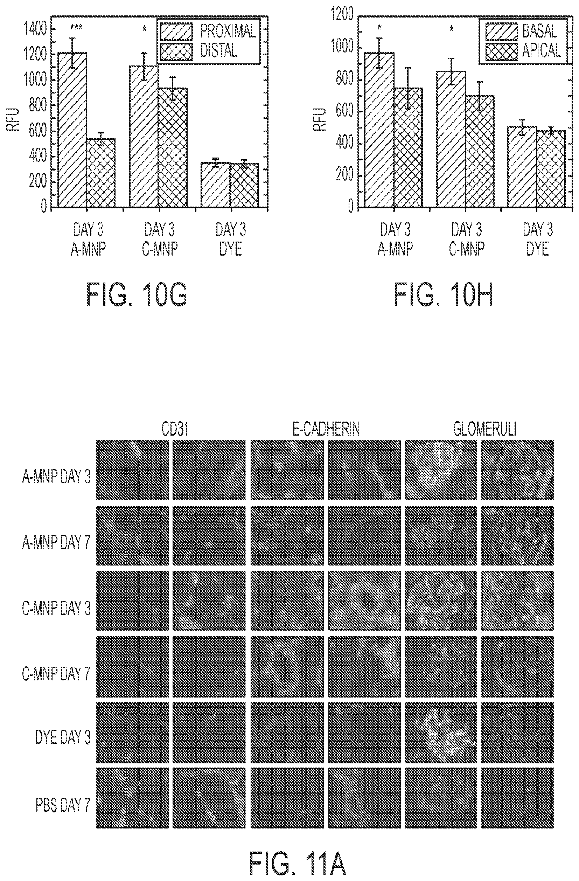

FIG. 10G shows fluorescence quantification in proximal versus distal tubules for each treatment (n=5, Mean ± SD).
FIG. 10H shows fluorescence quantification in the basal portion versus apical portion of tubule epithelial cells (n=5, Mean ± SD). ***: p<0.001; *: p<0.05. All scale bars denote 10 µm.
FIG. 11A shows representative micrographs of renal immunofluorescence. Immunofluorescence images were acquired from mouse tissues at day 3 and day 7 after MNP injection. Blue = DAPI stain for cell nuclei, red = fluorescence from nanoparticles (excluding bright red blood cells which exhibit uniform autofluorescence), and green = either CD31 (left 2 columns) or E-Cadherin staining (center 2 columns). Images from glomeruli are also shown (right 2 columns) with CD31 staining on the left and E-Cadherin staining on the right. Scale bar denotes 10 µm. (Note: The green signal intensities from CD31 and E-Cadherin stains were generated from different exposures and brightness levels, while all blue and green fluorescence signals were generated from identical exposures and brightness levels.)

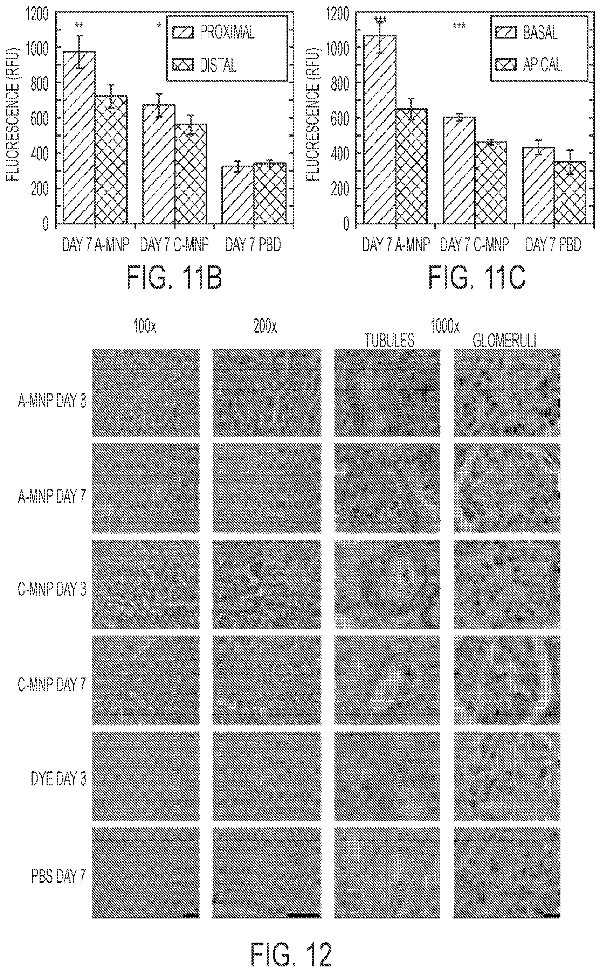

FIG. 11B shows fluorescence quantification in proximal versus distal tubules for each treatment at day 7.
FIG. 11C showsfluorescence quantification in the basal portion of tubule epithelial cells versus the apical membrane at day 7 (Mean ± SD). ***: p<0.001; **: p<0.01; *: p<0.05.
FIG. 12 shows immunohistochemical stains of renal tissue with anti-PEG antibody. Images of each group were taken with 10x, 20x, and 100x objectives (and 10x eyepieces) to generate images with the indicated magnification. Scale bars are 100 µm for 100x and 200x columns, 10 µm for 1000x column.

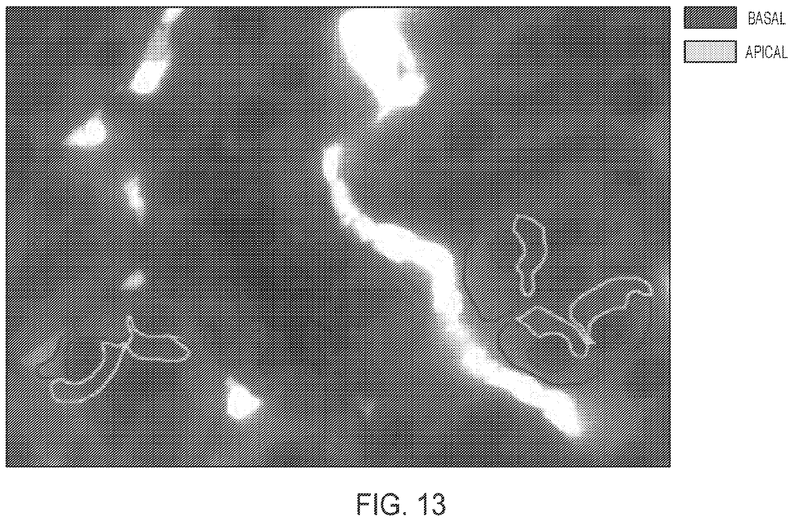

FIG. 13 shows graphic depicting selection of basal and apical portions of tubular epithelial cells for quantification in FIG. 10H , FIG. 11B, and FIG. 11C in ImageJ. The overlain sections were selected on the Cy5 channel images, and the representative image shown is that from FIG. 5A "Cationic NP" image.

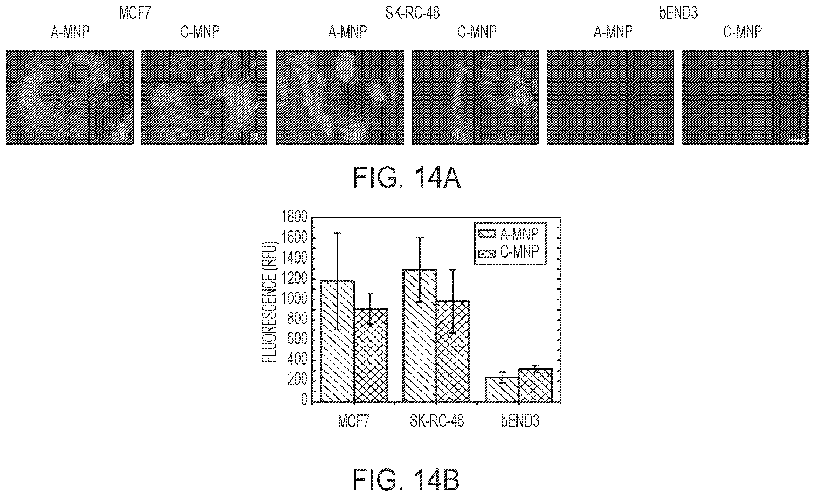

FIGS. 14A and 14B show nanoparticle uptake in cell lines.
FIG. 14A shows representative images of DEDC fluorescence in three cell lines after a 10 minute incubation with A-MNPs or C-MNPs.
FIG. 14B shows quantification of nanoparticle uptake in each cell line (Mean ± SD). Scale bar is 10 µm.

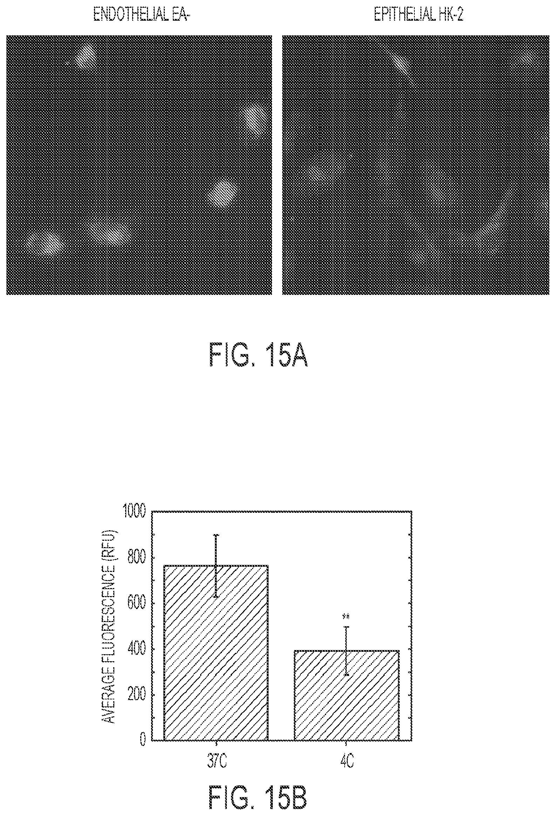

FIGS. 15A and 15B show nanoparticle uptake.
FIG. 15A shows that MNPs are taken up into the endothelial cell line EA-926 and the human kidney proximal tubular epithelial cell line HK-2.
FIG. 15B shows that nanoparticle uptake into HK-2 cells can be blocked by incubation at 4°C, indicating an energy-dependent uptake mechanism.

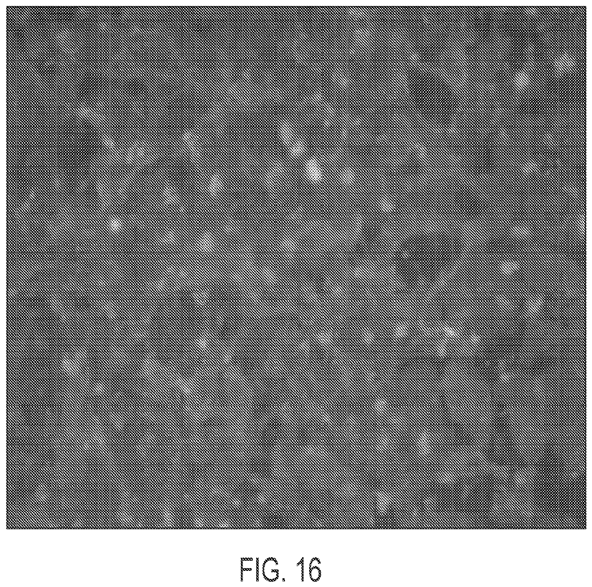

FIG. 16 shows nanoparticle uptake in human proximal tubular epithelial HK-2 cells. Red is fluorescence from nanoparticles, green is cell membrane dye.

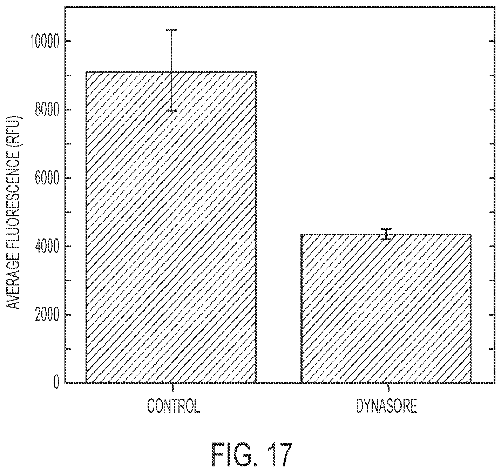

FIG. 17 shows MNP uptake into HK-2 cells can be inhibited by Dynasore, an inhibitor of clathrin/caveolin-mediated endocytosis.

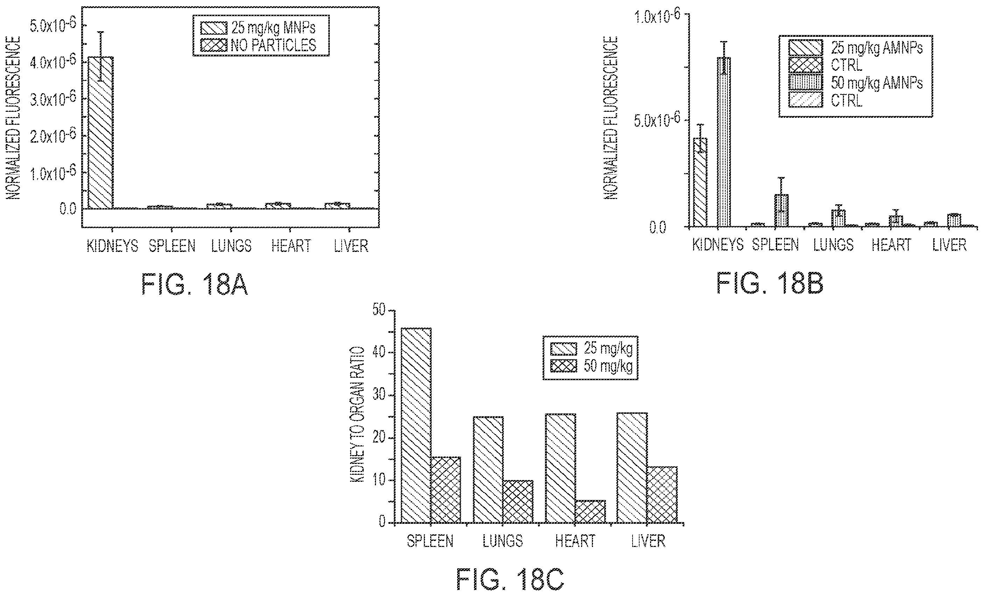

FIG. 18A shows *ex vivo* organ fluorescence from mice injected with 25 mg/kg MNPs or PBS normalized by total organ weight (Mean ± SD). Data shows up to 25-fold greater localization to the kidneys than any other organ.
FIG. 18B shows 25 mg/kg MNP injection versus 50 mg/kg. 25 mg/kp MNP injected reduced kidney targeting was by half, but almost complete reduction in non-specific targeting to other organs compared to 50 mg/kg MNP injection.
FIG. 18C shows specificity of MNPs to the kidneys compared to other organs comparing the two doses.

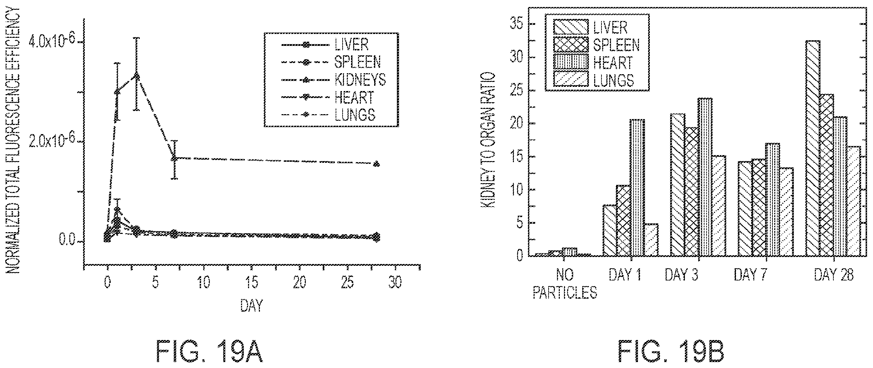

FIG. 19A shows biodistribution of 25 mg/kg MNPs over the course of 28 days. Results show specific renal accumulation persists for at least one month, and reaches its maximum at 7 days post-injection.
FIG. 19B shows specificity of 25 mg/kg MNPs to the kidney compared to other organs increases over time as non-specific accumulation is washed out.

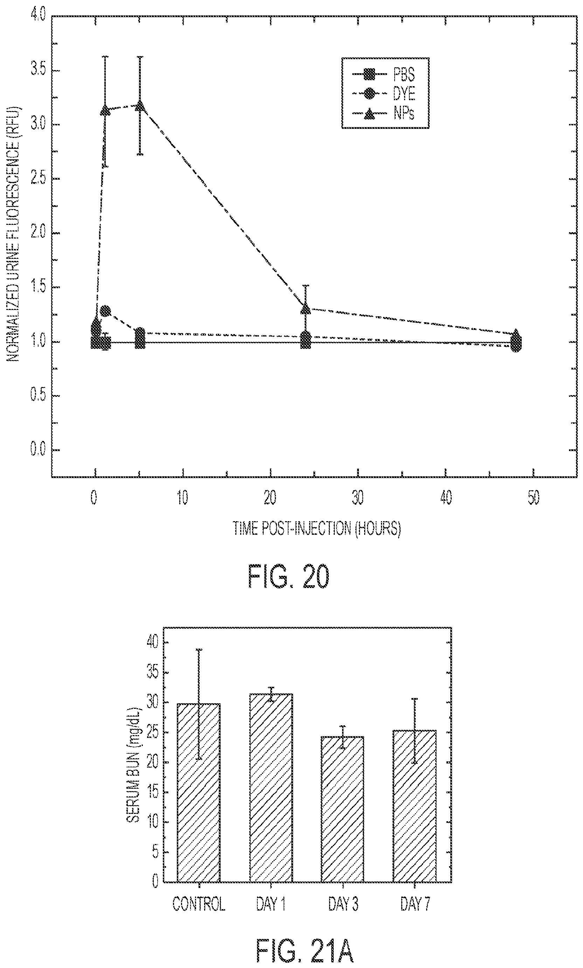

FIG. 20 shows fluorescent dye in the urine of mice injected with MNPs, PBS, or dye alone for 48 hours post-injection.
FIGS. 21A - 21D show renal health in mice injected with MNPs.

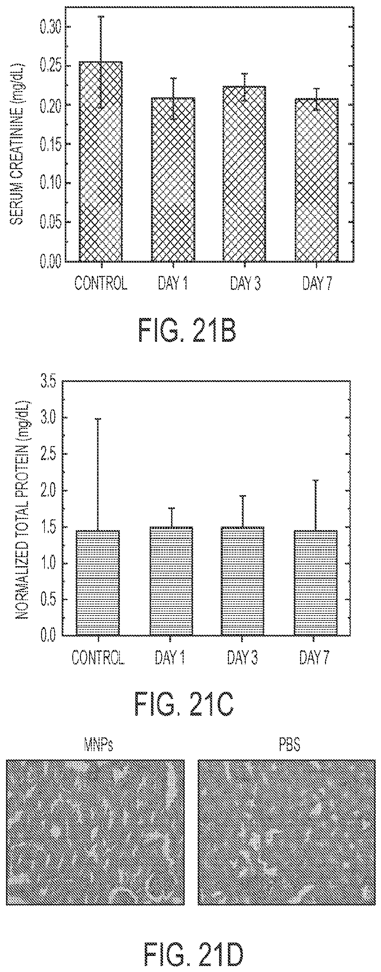

FIGS. 21A and 21B show serum nitrogen (FIG. 21A) and creatinine (FIG. 21B) of mice up to 7 days following nanoparticle administration.
FIG. 21C shows normalized total protein in the urine in mice treated with MNPs for up to 7 days following injection.
FIG. 21D shows representative H&E staining of mouse renal sections in mice treated with MNPs or PBS alone. These data suggest that selective localization of MNPs to kidneys does not affect renal function or cause any apparent toxicity.

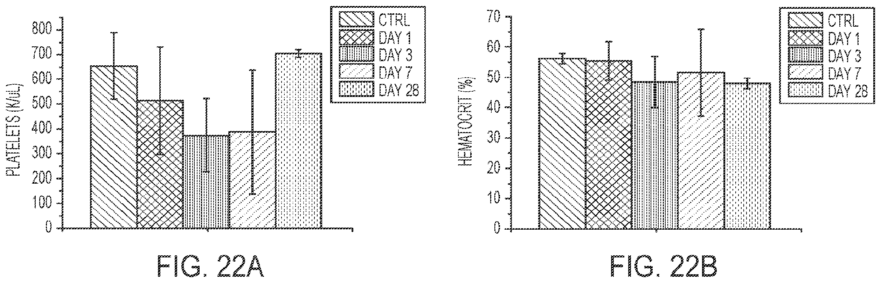

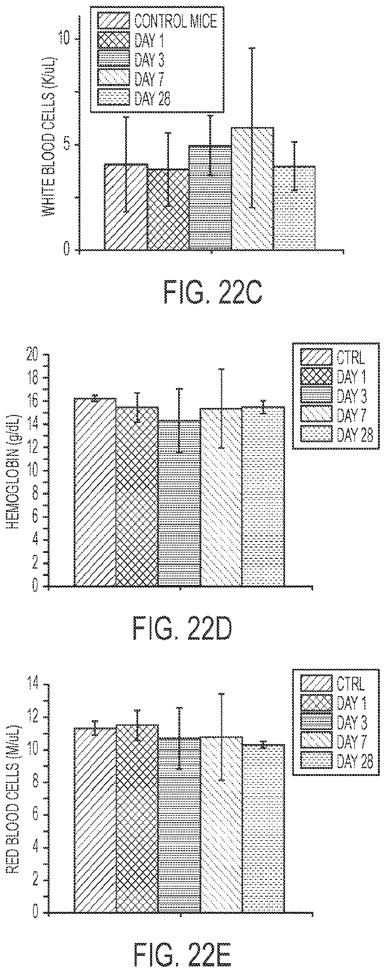

FIGS. 22A - 22E show that 25 mg/kg MNP injection shows no adverse effects on blood cells in mice.

### Detailed Description

Throughout the description, where compositions are described as having, including, or comprising specific components, or where methods are described as having, including, or comprising specific steps, it is contemplated that, additionally, there are compositions of the present invention that consist essentially of, or consist of, the recited components, and that there are methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any claim.

Described herein are synthesized "mesoscale" nanoparticles ("MNPs"), approximately 400 nm in diameter, which unexpectedly localized selectively in renal proximal tubules up at least 1.5 times more efficiently in the kidney than other organs. Furthermore, the nanoparticles described herein differ from those previously described primarily in size, PLGA size, PEG size, PEG functionality, and charge. Although nanoparticles typically localize in the liver and spleen, modulating their size and opsonization potential allowed for stable targeting of the kidneys through a new proposed uptake mechanism. Applying this kidney targeting strategy enables use in the treatment of renal disease and the study of renal physiology.

### Imaging agents

In certain embodiments, the compositions described herein include (i) imaging agents that are, or are associated with, the therapeutic agent, and/or (ii) imaging agents that are associated with, or are a part of, the MNPs. In certain embodiments, the imaging agents can include radiolabels, radionuclides, radioisotopes, fluorophores, fluorochromes, dyes, metal lanthanides, paramagnetic metal ions, superparamagnetic metal oxides, ultrasound reporters, x-ray reporters, and/or fluorescent proteins.

In certain embodiments, radiolabels comprise ^{99m}Tc, ¹¹¹In, ^{6a}Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹3N, ¹⁵O, ¹⁸F,¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, ⁸⁹Zr, and ¹⁹²Ir. In some embodiments, paramagnetic metal ions comprise Gd(III), Dy(III), Fe(III), and Mn(II). In some embodiments, Gadolinium (III) contrast agents comprise Dotarem, Gadavist, Magnevist, Omniscan, OptiMARK, and Prohance. In certain embodiments, x-ray reporters comprise iodinated organic molecules or chelates of heavy metal ions of atomic numbers 57 to 83.

In certain embodiments, PET (Positron Emission Tomography) tracers are used as imaging agents. In some embodiments, PET tracers comprise ⁸⁹Zr, ⁶⁴Cu, [¹⁸F] fluorodeoxyglucose.

In certain embodiments, fluorophores comprise fluorochromes, fluorochrome quencher molecules, any organic or inorganic dyes, metal chelates, or any fluorescent enzyme substrates, including protease activatable enzyme substrates. In some embodiments, fluorophores comprise long chain carbophilic cyanines. In other embodiments, fluorophores comprise DiI, DiR, DiD, and the like. Fluorochromes comprise far red, and near infrared fluorochromes (NIRF). Fluorochromes include but are not limited to a carbocyanine and indocyanine fluorochromes. In some embodiments, imaging agents comprise commercially available fluorochromes including, but not limited to Cy5.5, Cy5 and Cy7 (GE Healthcare); AlexaFlour660, AlexaFlour680, AlexaFluor750, and AlexaFluor790 (Invitrogen); VivoTag680, VivoTag-S680, and VivoTag-S750 (VisEn Medical); Dy677, Dy682, Dy752 and Dy780 (Dyomics); DyLight547, DyLight647 (Pierce); HiLyte Fluor 647, HiLyte Fluor 680, and HiLyte Fluor 750 (AnaSpec); IRDye 800CW, IRDye 800RS, and IRDye 700DX (Li-Cor); and ADS780WS, ADS830WS, and ADS832WS (American Dye Source) and Kodak X-SIGHT 650, Kodak X-SIGHT 691, Kodak X-SIGHT 751 (Carestream Health).

### Administration

Pharmaceutical compositions incorporating the MPs described herein may be administered according to any appropriate route and regimen. In some embodiments, a route or regimen is one that has been correlated with a positive therapeutic benefit.

In certain embodiments, the exact amount administered may vary from subject to subject, depending on one or more factors as is well known in the medical arts. Such factors may include, for example, one or more of species, age, general condition of the subject, the particular composition to be administered, its mode of administration, its mode of activity, the severity of disease; the activity of the specific MNP employed; the specific pharmaceutical composition administered; the half-life of the composition after administration; the age, body weight, general health, sex, and diet of the subject; the time of administration, route of administration, and rate of excretion of the specific compound employed; the duration of the treatment; drugs used in combination or coincidental with the specific compound employed and the like. Pharmaceutical compositions may be formulated in dosage unit form for ease of administration and uniformity of dosage. It will be understood, however, that the total daily usage of the compositions will be decided by an attending physician within the scope of sound medical judgment.

Compositions described herein may be administered by any route, as will be appreciated by those skilled in the art. In certain embodiments, compositions described herein are administered by oral (PO), intravenous (IV), intramuscular (IM), intra-arterial, intramedullary, intrathecal, subcutaneous (SQ), intraventricular, transdermal, interdermal, intradermal, rectal (PR), vaginal, intraperitoneal (IP), intragastric (IG), topical (*e.g*., by powders, ointments, creams, gels, lotions, and/or drops), mucosal, intranasal, buccal, enteral, vitreal, sublingual; by intratracheal instillation, bronchial instillation, and/or inhalation; as an oral spray, nasal spray, and/or aerosol, and/or through a portal vein catheter.

In some embodiments, the pharmaceutical compositions and/or MNPs thereof may be administered intravenously (e.g., by intravenous infusion), by intramuscular injection, by intratumoural injection, and/or via portal vein catheter, for example. However, the subject matter described herein encompasses the delivery of pharmaceutical compositions and/or MNPs thereof in accordance with embodiments described herein by any appropriate route taking into consideration likely advances in the sciences of drug delivery.

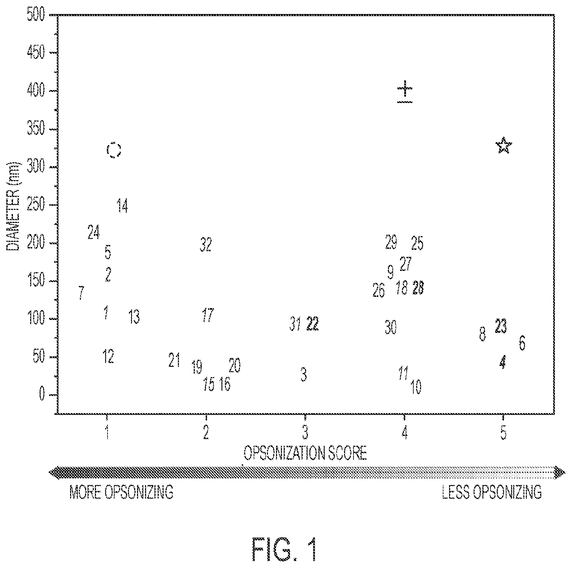

To investigate the parameters that may influence nanoparticle localization, the literature was probed to construct a simple plot to consolidate nanoparticle localization data from multiple studies as shown in FIG. 1. The references are listed in Table 2. Major organs were noted as to which nanoparticles localized as well as the nanoparticle size and the relative degree to which the particle may be opsonized by serum proteins-a natural process that labels exogenous materials for phagocytic destruction by the Mononuclear Phagocyte System (MPS). Avoidance of MPS-mediated phagocytosis was achieved by nanoparticles with "stealth", or non-opsonizing, materials such as polyethylene glycol (PEG) or natural lipoproteins. According to this survey of the literature, the majority of untargeted nanoparticles primarily accumulate in the liver or spleen and the selective targeting to other organs, including the kidneys or lymph nodes, is rare, although it appears to require a relatively low-opsonizing surface chemistry.

It is found herein that nanomaterial size has a demonstrated effect on biodistribution. Certain synthetic polymers, low-molecular weight proteins, and peptides less than 20 kDa in molecular weight exhibit renal tubule biodistribution but are quickly cleared from the body. Nanoparticles less than 250 nm tend to accumulate in the liver or spleen, either through MPS trafficking or entrance through liver fenestrations (approximately 100 nm) as shown in FIG. 1. Microparticles (e.g., particles with diameters above 1000 nm) often localize in the lungs due to entrapment in pulmonary capillary beds. Mesoscale nanoparticles refer to the larger gamut of nanoparticles above 100 nm in diameter. To date, the long-term biodistribution and tissue localization of mesoscale nanoparticles greater than 250 nm have not been studied in depth.

Herein, mesoscale nanoparticles (MNPs) were synthesized to avoid MPS organs and to selectively and stably accumulate in the kidneys up to seven times more efficiently than other organs. The parameter space required for this localization in terms of particle size and opsonization potential was determined. The nanoparticles accumulated in proximal versus distal renal tubules and more so at their basal rather than the apical membranes. Without being bound by theory, this specific accumulation may be due to a mechanism in which MNPs are endocytosed by endothelial cells of the peritubular capillaries because of the pressure drop in the nephron and the large absorptive pressure of the capillaries. Applying this targeting strategy, mesoscale nanoparticles can be used to treat diseases that affect the proximal tubules of the kidneys and to study renal blood flow.

Anionic (A-MNPs) and cationic (C-MNPs) coated with poly(lactic-co-glycolic acid) (PLGA) functionalized with PEG were synthesized and measured to be approximately 400 nm in diameter. The nanoparticles were loaded with a fluorescent dye for biodistribution studies. Carboxylic acid-terminated PLGA was conjugated to heterobifunctional amine-PEG-carboxylic acid. 1H NMR was performed to confirm conjugation. The nanoprecipitation method was used to form A-MNPs of 386.7 nm in diameter with a ζ potential of -19.5 mV as determined by dynamic light scattering (DLS) and electrophoretic light scattering (ELS) and is shown in Table 1, FIG. 5A. As depicted in FIGS. 5A and 5B, size and spherical morphology by scanning electron microscopy (SEM) was confirmed. Discrepancy in sizes as measured by SEM and DLS are attributable to shrinkage of the polymer upon drying and differences between dry and hydrodynamic diameters, the latter of which is measured by DLS. The diameter of the mesoscale nanoparticles did not change in size over the course of 48 hours in serum. Didodecyldimethylammonium bromide (DMAB) was introduced to form C-MNPs of 402.8 nm diameter and a ζ potential of 18.3 mV. Both particle formulations encapsulated 2.2 µg 3,3'-diethylthiadicarbocyanine iodide (DEDC) fluorescent dye per 1 mg of nanoparticles. Additionally, the total fluorescence from each nanoparticle formulation was essentially identical as shown in FIG. 5B.

In order to explore nanoparticle stability, aggregation, dye release, and ζ potential assays were performed. Each particle formulation exhibited similar stability after incubation in PBS for three days as shown by a particle size as shown in FIG. 3C). Both nanoparticles were stable in storage conditions for at least three days as measured in PBS, and in serum for up to two days for 48 hours in 100% fetal bovine serum (FBS), at which point they began to aggregate. Upon introducing FBS, both cationic and anionic nanoparticles reached approximately the same ζ potential as shown in FIG. 3D). Finally, dye release assays showed similar release kinetics in PBS and FBS as shown in FIG. 3E.

Table 1 shows size (DLS) and surface charge (zeta potential) data of dye-loaded mesoscale nanoparticles.

**Table 1**

| **Nanoparticle** | **Diameter** | **ζ Potential** |
|---|---|---|
| Anionic mesoscale nanoparticle (A-MNP) | 386.7 ± 18.7 nm | -19.5 ± 0.6 mV |
| Cationic mesoscale nanoparticle (C-MNP) | 402.8 ± 23.4 nm | 18.3 ± 1.3 mV |

The nanoparticles selectively accumulated in the kidneys of mice following intravenous injection. 50 mg/kg of A- or C-MNPs was intravenously injected into female SKH-1 mice and imaged the animals daily for up to 7 days and bi-weekly thereafter for approximately 3 months. Biodistribution was measured by fluorescence *in vivo* in order to track nanoparticle localization and degradation over time, a widely-used method that closely approximates other biodistribution assays.

Neither mice treated with anionic (A-MNP) nor cationic mesoscale nanoparticles (C-MNPs) exhibited a significant weight change at day 3 or day 7 compared to dye alone-treated mice after 3 days as shown in FIG. 4A. There was a significant weight increase in mice treated with PBS control at day 7 compared to dye alone. Therefore, any negative effects on weight of nanoparticles may also be attributed to the dye, suggesting that MNPs themselves do not induce additional toxicity. The kidneys of mice treated with either MNP formulation showed no histomorphological evidence of damage after 3 or 7 days as shown in FIG. 4B. Haematoxylin/eosin stained slides were reviewed by a board-certified anatomic pathologist.

Nanoparticles localized to both kidneys and the chest region as shown in FIG. 5A and FIGS. 6A and 6B. Mice treated with either A- or C-MNPs exhibited brighter fluorescence from the kidneys than mice treated with PBS alone or dye alone at all time points as shown in FIG. 6A. Fluorescence decreased rapidly until approximately the third day following injection and fluorescence decreased slowly thereafter. Renal fluorescence was visible for approximately 2 months following injection as shown in FIG. 6B. This pattern of nanoparticle degradation appears consistent with other reports of slow *in vivo* PLGA degradation.

Upon organ extraction, to obtain more quantitative biodistribution patterns, the fluorescence signal was significantly greater in the kidneys than in any other organ analyzed as shown in FIG. 5B. The fluorescence emission from the kidneys was greatest at day 3 for C-MNP-treated mice: 5.3 times greater than the next-highest organ, the heart, for A-MNPs and 5.9 times for C-MNPs at day 3. Fluorescence was also greatest in the kidneys at day 7: 4.5 times greater for A-MNPs and 3.7 times greater than the heart for C-MNPs. Combined fluorescence and computed tomography (CT) imaging focused solely on the kidneys of a mouse treated with A-MNPs confirmed kidney localization as well as relatively even distribution throughout the kidneys as shown in FIG. 5C and FIG. 5D. Thus, surface charge did not significantly affect the biodistribution of these nanoparticles, which may be explained by the finding that incubation in FBS caused the ζ potentials of A-MNP and C-MNP nanoparticles to become very similar as shown in FIG. 5D.

The biodistribution of neutral (ζ potential = 0.38mV) mesoscale nanoparticles (N-MNPs), functionalized with methoxy-PEG (mPEG), was also measured and is shown in FIGS. 7A and 7B. These particles, 328.1 nm in diameter, similarly localized preferentially in the kidneys and exhibited 6.7 times more fluorescence than the next-brightest organ, the lungs. Neutral PLGA mesoscale nanoparticles (N-MNPs) functionalized with a methoxy PEG surface were synthesized to encapsulate 0.23 µg of DEDC dye per 1 mg of nanoparticle. The diameter of the resulting nanoparticles averaged 328.1 ± 5.3 nm with a ζ potential of 0.38 ± 0.70 mV. *In vivo,* they showed a similar biodistribution profile to both A- and C-MNPs. N-MNP accumulation in the kidneys was far greater than in other organs as depicted in FIG. 7A. *Ex vivo* kidney fluorescence was 6.7 times higher than the heart, the second brightest organ as depicted in FIG. 7B.

As shown in FIGS. 8A and 8B, surface PEGylation is necessary for kidney localization by determining the biodistribution of non-PEGylated PLGA opsonizing nanoparticles (O-MNPs) with a diameter of 327.1 nm and an anionic surface (-18.1 mV). These particles primarily localized to the liver 30 minutes following intravenous injection and appeared to clear by hepatobiliary excretion at 4 hours, which was confirmed by the nanoparticle localization in the large intestine at this time point. This result correlates with previous research demonstrating that opsonizing nanoparticles are endocytosed by Kupffer cells within the liver within seconds of injection. Little organ fluorescence was detected above background after 1 day and none after 3 days; thus, surface PEGylation is beneficial for long-term degradation and controlled payload release from the particles. Therefore, MNP kidney targeting appears to depend predominantly on size and surface functionalization, but is independent of moderate surface charges.

Opsonizing PLGA nanoparticles without PEG (O-MNPs), loaded with IR783 dye, localized in the liver 30 minutes following injection as shown in FIG. 8A. Four hours following injection, the signal in the liver disappeared while low-level signal appeared in the intestines, suggesting hepatobiliary clearance of the nanoparticles. This experiment is consistent with studies showing that PLGA nanoparticles without PEG or other "stealth" moieties are endocytosed by liver Kupffer cells shortly after injection. Total fluorescence dissipated by 1 day post-injection and was absent at 3 days as shown in FIG. 8B. This experiment supports a model in which a non-opsonizing surface is necessary to avoid rapid uptake by the Mononuclear Phagocyte System (MPS) allowing targeting of non-MPS organs.

Fluorescence imaging in live mice underestimates signal in the kidneys, heart, and other dense tissues compared to *ex vivo* quantification.

The difference in nanoparticle fluorescence emission intensity between organs *in vivo* and *ex vivo* was assessed. One mouse, treated with 50 mg/kg A-MNPs, was euthanized 2 days following injection. *In vivo,* prior to euthanization, the fluorescence localization pattern was identical to that shown in similar experiments with the same treatment and is shown in FIG. 3A and FIG. 6B. Following euthanization and organ extraction, the carcass lacked the bright foci, confirming that the fluorescence emanated from the removed kidneys as shown in FIG. 9A. Furthermore, the fluorescence in the kidneys *ex vivo* was brighter than all other organs, as previously seen in FIG. 3B with these nanoparticles. Additionally, the *ex vivo* signal from each kidney was 25-30 times higher than the signal *in vivo,* but this phenomenon was not seen in the lungs, which was only 2.0-3.5 times higher *ex vivo* than *in vivo* as decpicted in FIG. 9B. To investigate this difference, the mouse carcass was imaged, as shown in FIG. 9A, following organ removal, which revealed a significant decrease in fluorescent foci discussed above. As shown in FIG. 9B, the extent to which *in vivo* imaging underestimates kidney fluorescence is 25-30 times, compared to 2.0-3.5 times for the lungs.

In order to provide higher order spatial information regarding the distribution of nanoparticles in the kidney, both immunofluorescence (IF) and immunohistochemistry (IHC) techniques was used. Kidney tissue from treated or control mice was sectioned and stained for CD31 (blood vessels) or E-cadherin (distal tubules) expression by IF and for the presence of PEG by IHC. Nanoparticle fluorescence in the renal tubules of MNP-treated mice was significantly higher than negative controls as shown in FIGS. 10A - 10C and FIGS. 11A - 11C. Furthermore, the fluorescence was brighter in proximal tubules compared to distal tubules as revealed by costaining for E-cadherin, a marker of distal tubules as shown in FIG. 10A, 10B, 10G, and FIGS. 11A - 11C. This tissue distribution pattern was confirmed by antibody staining for PEG as shown in FIG. 10E and FIG. 12. Thus, co-localized fluorescence from MNPs and staining for the nanoparticle surface confirmed that both the polymer and the encapsulated dye cargo are present in the proximal tubules. Interestingly, there was negligible particle localization in the endothelium or mesangial c10Aells in the glomeruli as determined by IF and IHC as shown in FIG. 10D, 10F, FIGS. 9A and 9B, and FIG. 12. The fluorescence staining intensity was greater at the basolateral side of the epithelial cells as depicted in FIGS. 8A and 8B, FIGS. 10C, 10H, and FIGS. 11A - 11C. *In vivo* fluorescence dissipated over time as shown in FIGS. 6A and 6B, indicating that MNPs target and release payload in a controlled manner in the proximal tubules. Immunofluorescence and immunohistochemistry imaging of mouse renal tissues from mice treated with A- or C-MNPs at day 7 revealed a similar basal staining pattern in the proximal tubules as at day 3 as shown in FIGS. 11A, 11B, 11C, FIG. 13, and FIG. 12).

The parameters necessary to effect localization toward the kidneys and away from other organs such as the liver and spleen were investigated. The role of nanoparticle size with respect to the organ of localization is not clear. Also, it is apparent from this study that a relatively small surface charge, or lack thereof, does not significantly affect biodistribution because A-MNPs, C-MNPs, and N-MNPs all localized in the kidneys. These findings, in agreement with the literature as shown in FIG. 1, suggest that an important factor for directing nanoparticles to organs other than the liver and spleen is a relatively non-opsonizing surface. To this end, Owens and Peppas have suggested that longer PEG chains (>2000 Da) are the most effective at reducing opsonization. Additionally, PEG surface coverage greater than 2% is important. MNPs have 5000 Da PEG chains and a PEG/PLGA weight ratio of 9-13%, suggesting that they will significantly reduce opsonization. The MNPs disclosed herein have a similar PEG chain size with PEG/PLGA ratios in approximately the same range as others in the literature as shown in FIG. 1.

Next, the mechanism of localization to the kidney at the tissue level was probed. Histology confirmed that MNPs predominantly localized in the basolateral region of proximal tubule epithelial cells. Previous work showed that nanoparticles of approximately 80 nm in diameter with a non-opsonizing surface targeted the kidney glomeruli. The fenestrations of this segment of the nephron (approximately 80-100 nm) are too small for the MNPs described herein to pass through, however. There are also fenestrations in the peritubular capillaries which run along the renal tubules; however those are also reportedly too small (~5 nm) for passage of MNPs. Thus, MNPs must be endocytosed by endothelial cells of the peritubular capillaries, which is confirmed *in vitro* and depicted in FIG. 12. It is likely that MNPs are endocytosed to a greater extent by peritubular endothelial cells than glomerular endothelial cells due to the sharp drop in pressure in this segment of the nephron (e.g., 50 mm Hg to 10 mm Hg) and the large absorptive pressure of peritubular capillaries which allows the particles greater opportunity to interact with capillary endothelial cell membranes. Not to be bound by theory, this data suggests that MNPs are transcytosed across the thin (e.g., less than 500 nm) cells and released into the tubulointerstitium between the capillary and epithelial cells of the tubule. The MNPs would likely then be endocytosed by epithelial cells of the tubule as shown in FIGS. 14A and 14B. Fluorescence microscopy analysis revealed uptake of MNPs in all three cell lines observed as shown in FIG. 14A. While SK-RC-48 human clear cell renal cell carcinoma and MCF7 human breast adenocarcinoma cells exhibited strong emission, the bEND3 mouse brain endothelial cells also took up nanoparticles, but at a lower rate as shown in FIG. 14B. Cells were also observed for approximately 48 hours following treatment and no significant cytotoxicity or loss of fluorescence was observed. In these renal epithelial cells the nanoparticles were retained for days to weeks in mice before degradation. Previous work has shown that PLGA nanoparticles avoid endo-lysosomal degradation and become associated with the endoplasmic reticulum and Golgi after endocytic uptake, potentially increasing the utility of MNPs for drug delivery in the treatment of diseases affecting the proximal tubules.

The present disclosure describes synthesis and characterization of a class of polymeric mesoscale nanoparticles which selectively and stably localized in the proximal tubule epithelium of the kidneys. Exploring the parameter spaces responsible for kidney targeting revealed that low opsonization potential is important for kidney targeting, but moderate changes in zeta potential had no effect on localization. This data suggests a mechanism of localization to the proximal tubules supported by histological evidence whereby the nanoparticles are endocytosed by endothelial cells of the peritubular capillaries because of the pressure drop in the nephron and the large absorptive pressure of the capillaries. This targeting strategy can be applied to the treatment of diseases which affect the proximal tubules and as a tool for studying renal physiology.

### Experimental Examples

### Nanoparticle biodistribution literature survey

A brief literature survey was conducted to investigate the effects of nanoparticle size and opsonization potential on biodistribution. Publications that studied nanoparticle biodistribution without the use of molecular targeting moieties were selected. Studies performed in diseased animals were excluded in order to determine biodistribution in healthy, uncompromised mice or rats. Only nanoparticles administered intravenously were included. The nanoparticle diameter, surface functionalization, and primary site of localization were recorded from each paper. Of publications disclosing many nanoparticles with minor size iterations, one representative particle was chosen. The opsonization potential of each particle was assigned a score with 1 being the most opsonizing and 5 being the least opsonizing.

Category binning was performed as follows: 1- PLGA or gold particles with no coating; 2- Relatively opsonizing proteins, small molecule labels, or polymers; 3- Relatively non-opsonizing protein or polymer (as described in the original manuscript); 4- PEG-coated particles with surface charge -15 < X > +15; 5- PEG-coated particles with surface charge -15 > X < +15 or non-opsonizing lipid. Each cited nanoparticle was assigned a number as shown in Table 2. The size and assigned degree of opsonization (e.g., surface category) were plotted for each referenced nanoparticle as shown in FIG. 1. The nanoparticles disclosed herein are the last four entries listed at the bottom of the table.

Table 2 shows an exemplary nanoparticle distribution literature survey.

**Table 2**

| Type of Particle | Surface Coating | Primary Localization | Size (nm) | Opsonization Score | Particle # | Ref.[ #] |
|---|---|---|---|---|---|---|
| PLGA | None | Spleen | 105 | 1 | 1 | 1 |
| PLGA | None | Spleen | 160 | 1 | 2 | 1 |
| Gold-Apotransferrin | Apotransferrin | Liver | 5 | 3 | 3 | 2 |
| Lipid/Protein | Apolipo-protein | Kidney | 25 | 5 | 4 | 3 |
| PLGA | None | Liver | 187 | 1 | 5 | 4 |
| PLGA | mPEG 5000 | Liver | 67 | 5 | 6 | 5 |
| PLGA | None | Liver | 134 | 1 | 7 | 5 |
| Gold50 | mPEG 5000 | Liver | 79 | 5 | 8 | 6 |
| PLGA | PEG 3400 | Liver | 150 | 4 | 9 | 7 |
| Gold | PEG | Liver | 10 | 4 | 10 | 8 |
| Gold | PEG | Spleen | 30 | 4 | 11 | 8 |
| Gold | None | Liver | 50 | 1 | 12 | 9 |
| Gold | None | Liver | 100 | 1 | 13 | 9 |
| Gold | None | Liver | 250 | 1 | 14 | 9 |
| Silica | Radiolabel | Spleen | 18 | 2 | 15 | 10 |
| Silica | Fluorescent Dye | Liver | 18 | 2 | 16 | 10 |
| Iron Oxide | Starch | Spleen | 104 | 2 | 17 | 11 |
| Iron Oxide | mPEG | Spleen | 142 | 4 | 18 | 11 |
| Iron Oxide | mPEG | Spleen | 169 | 4 | 19 | 11 |
| Vinylpyrrolidone 100: N-isopropylacrylamide 0 | None | Liver | 35 | 2 | 20 | 12 |
| Vinylpyrrolidone 50: N-isopropylacrylamide 50 | None | Liver | 45 | 2 | 21 | 12 |
| Radio labeled chitosan | Chitoson | Stomach | 70 | 3 | 22 | 13 |
| PLA | mPEG | Lymph Node | 70 | 5 | 23 | 14 |
| PLGA | None | Liver | 214 | 1 | 24 | 15 |
| PLGA | mPEG 5000 | Liver | 198 | 4 | 25 | 15 |
| PLA | Poloxamer 188 | Liver | 136 | 4 | 26 | 16 |
| PLA | PEG20k (10%) | Liver | 176 | 4 | 27 | 16 |
| PBAE | Pluronic F108 | Kidney | 113 | 4 | 28 | 17 |
| PCL | Pluronic F108 | Liver | 200 | 4 | 29 | 17 |
| Doxil (Liposome) | PEG | Liver | 87 | 4 | 30 | 18 |
| Iron Oxide | Pluronic F127 | Spleen | 86 | 3 | 31 | 19 |
| Lipid | Lecithin | Spleen | 197 | 2 | 32 | 20 |
| PLGA | PEG-carboxylic acid | Kidney | 386 | 4 | (-) | Described herein |
| PLGA | PEG-carboxylic acid-DMAB | Kidney | 432 | 4 | (+) | Described herein |
| PLGA | methoxy PEG | Kidney | 328 | 5 | (★) | Described herein |
| PLGA | None | Hepatobiliary | 327 | 1 | ( 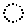 ) | Described herein |

### Ref.

[1] Yadav, K. S.; Chuttani, K.; Mishra, A. K.; Sawant, K. K. PDA Journal of Pharmaceutical Science and Technology 2011, 65, 131-139.
[2] Sun, C.; Yang, H.; Yuan, Y.; Tian, X.; Wang, L.; Guo, Y.; Xu, L.; Lei, J.; Gao, N.; Anderson, G. J. J. Am. Chem. Soc. 2011, 133, 8617-8624.
[3] Fischer, N. O.; Weilhammer, D. R.; Dunkle, A.; Thomas, C.; Hwang, M.; Corzett, M.; Lychak, C.; Mayer, W.; Urbin, S.; Collette, N. PloS one 2014, 9, e93342.
[4] Tosi, G.; Vergoni, A.; Ruozi, B.; Bondioli, L.; Badiali, L.; Rivasi, F.; Costantino, L.; Forni, F.; Vandelli, M. J. Controlled Release 2010, 145, 49-57.
[5] Avgoustakis, K.; Beletsi, A.; Panagi, Z.; Klepetsanis, P.; Livaniou, E.; Evangelatos, G.; Ithakissios, D. Int. J. Pharm. 2003, 259, 115-127.
[6] Choi, C. H. J.; Zuckerman, J. E.; Webster, P.; Davis, M. E. Proc. Natl. Acad. Sci. 2011, 108, 6656-6661.
[7] Dhar, S.; Kolishetti, N.; Lippard, S. J.; Farokhzad, O. C. Proc. Natl. Acad. Sci. 2011, 108, 1850-1855.
[8] Zhang, X.-D.; Wu, D.; Shen, X.; Liu, P.-X.; Yang, N.; Zhao, B.; Zhang, H.; Sun, Y.-M.; Zhang, L.-A.; Fan, F.-Y. International journal of nanomedicine 2011, 6, 2071.
[9] De Jong, W. H.; Hagens, W. I.; Krystek, P.; Burger, M. C.; Sips, A. J.; Geertsma, R. E. Biomaterials 2008, 29, 1912-1919.
[10] Kumar, R.; Roy, I.; Ohulchanskky, T. Y.; Vathy, L. A.; Bergey, E. J.; Sajjad, M.; Prasad, P. N. ACS nano 2010, 4, 699-708.
[11] Cole, A. J.; David, A. E.; Wang, J.; Galbán, C. J.; Yang, V. C. Biomaterials 2011, 32, 6291-6301.
[12] Gaur, U.; Sahoo, S. K.; De, T. K.; Ghosh, P. C.; Maitra, A.; Ghosh, P. Int. J. Pharm. 2000, 202, 1-10.
[13] Banerjee, T.; Mitra, S.; Kumar Singh, A.; Kumar Sharma, R.; Maitra, A. Int. J. Pharm. 2002, 243, 93-105.
[14] Chaney, E. J.; Tang, L.; Tong, R.; Cheng, J.; Boppart, S. A. Molecular imaging 2010, 9, 153.
[15] Li, Y.-P.; Pei, Y.-Y.; Zhang, X.-Y.; Gu, Z.-H.; Zhou, Z.-H.; Yuan, W.-F.; Zhou, J.-J.; Zhu, J.-H.; Gao, X.-J. J. Controlled Release 2001, 71, 203-211.
[16] Mosqueira, V. C. F.; Legrand, P.; Morgat, J.-L.; Vert, M.; Mysiakine, E.; Gref, R.; Devissaguet, J.-P.; Barratt, G. Pharmaceutical research 2001, 18, 1411-1419.
[17] Shenoy, D.; Little, S.; Langer, R.; Amiji, M. Pharmaceutical research 2005, 22, 2107-2114.
[18] Bao, A.; Goins, B.; Klipper, R.; Negrete, G.; Phillips, W. T. J. Pharmacol. Exp. Ther. 2004, 308, 419-425.
[19] Jain, T. K.; Reddy, M. K.; Morales, M. A.; Leslie-Pelecky, D. L.; Labhasetwar, V. Mol. Pharm. 2008, 5, 316-327.
[20] Yang, S. C.; Lu, L. F.; Cai, Y.; Zhu, J. B.; Liang, B. W.; Yang, C. Z. J. Controlled Release 1999, 59, 299-307.

### Nanoparticle synthesis, characterization, and biodistribution studies:

Poly(lactic-co-glycolic acid) conjugated to polyethyle glycol (PLGA-PEG) was synthesized similarly to previously described methods as described by Cheng, J.; Teply, B. A.; Sherifi, I.; Sung, J.; Luther, G.; Gu, F. X.; Levy-Nissenbaum, E.; Radovic-Moreno, A. F.; Langer, R.; Farokhzad, O. C. Biomaterials 2007, 28, 869-876. Carboxylic acid-terminated poly(lactic-co-glycolic acid) (PLGA) (50:50; MW 38-54 kDa) (90-130 µmol) (Aldrich; St. Louis, MO) was dissolved in 10 mL methylene chloride and activated with N-hydroxysuccinimide (NHS, 135 mg, 1.2 mmol) (Aldrich) and 1-ethyl-3-(3-dimethylaminopropyl)-carbodiimide (EDC, 230 mg, 1.2 mmol) for 30 minutes with stirring. Conjugated PLGA-NHS was precipitated with 5 mL ethyl ether, washed 3x with cold 50:50 ethyl ether:methanol, and dried under vacuum. To PLGA-NHS (1g, 18-26 µmol) in 4 mL chloroform, 250 mg (50 µmol) NH2-PEG-COOH (MW 5 kDa) (Nanocs; New York, NY) was added with 28 mg (220 µmol) N,N-diisopropylethylamine. After the reaction proceeded with mixing overnight, conjugated PLGA-PEG was precipitated and washed 3x with 5 mL cold methanol and allowed to dry under vacuum. The polymer was characterized by 1H NMR as previously described in Cheng, J.; Teply, B. A.; Sherifi, I.; Sung, J.; Luther, G.; Gu, F. X.; Levy-Nissenbaum, E.; Radovic-Moreno, A. F.; Langer, R.; Farokhzad, O. C. Biomaterials 2007, 28, 869-876.

PLGA-PEG was used to form anionic mesoscale nanoparticles (A-MNPs) by the nanoprecipitation method. PLGA-PEG (100 mg) was dissolved with the fluorescent Cy5 mimic 3,3'-Diethylthiadicarbocyanine iodide (DEDC) (Acros Organics; Geel, Belgium) (10mg) in 2 mL acetonitrile. This solution was added dropwise to 4 mL water with 100 µL 10% Pluronic F-68 (Gibco; Grand Island, NY) and stirred for 2 hours. The solution was then centrifuged for 15 minutes at 6,600 RPM, washed, and centrifuged again. Particles were lyophilized in a 2% sucrose solution for storage at -20°C. Dried particles were suspended in phosphate-buffered saline (PBS) or water and analyzed for size by dynamic light scattering (DLS) (Malvern; Worcestershire, United Kingdom) and scanning electron microscopy (SEM) following gold-palladium coating (Zeiss; Oberkochen, Germany), for ζ potential by electrophoretic light scattering (ELS) (Malvern), and for encapsulation by UV-VIS absorbance (Jasco; Easton, MD) and DEDC fluorescence (Tecan; Mannedorf, Switzerland). To synthesize cationic mesoscale nanoparticles (C-MNPs), 9.5 mg lyophilized A-MNPs were suspended in a solution of 0.5 mg/mL didodecyldimethylammonium bromide (DMAB). This method can modify an anionic PLGA-PEG nanoparticle surface, resulting in cationic nanoparticles functionally shown to be stable for *in vitro* and *in vivo* applications. Before use, both A-MNPs and C-MNPs were centrifuged and resuspended in PBS to remove unincorporated dye and/or DMAB.

All experiments performed in animals were approved by and carried out in accordance with the MSKCC Institutional Animal Care and Use Committee. Female 4-8 week SKH-1 Elite hairless mice (Crl:SKH1-Hrhr) (Charles River; Troy, NY) were used in order to reduce background autofluorescence or absorbance from haired mice. They were fed irradiated 5V75 alfalfa-free food (LabDiet; St. Louis, MO) to reduce fluorescent background in imaging. Groups of 6 mice each were injected intravenously via the tail vein with 50 mg/kg of A-MNPs or C-MNPs encapsulating DEDC. Control groups of 4 mice each were injected with 100 µL PBS or 23 µg/kg DEDC in PBS with 0.5% DMSO (equal to the amount of encapsulated dye in particle-injected mice). Mice were imaged dorsally with an IVIS Spectrum Pre-clinical *In vivo* Imaging System (Perkin Elmer; Waltham, MA) using 640 nm excitation and 680 nm emission filters to determine fluorescence biodistribution at the following times post-injection: 30 minutes, 4 hours, and 1-7 days. *In vivo* fluorescence images were analyzed using Living Image Software v4.3 (Perkin Elmer) with regions of interest (ROIs) selected around each kidney and the central lung region to obtain total fluorescence efficiency (TFE) from each. On day 3, 3 mice from the A-MNP and C-MNP groups, 2 mice from the PBS control group, and all 4 mice from the DEDC control group were euthanized by carbon dioxide overdose. The following organs were harvested and imaged for fluorescence: heart, lungs, kidneys, liver, and spleen. TFE for each organ was obtained and normalized by organ weight to obtain organ-level biodistribution. Normalized organ fluorescence for each group was averaged and standard deviations obtained. The organs were weighed and fixed with 4% paraformaldehyde (PFA) overnight at 4°C. One mouse treated with anionic nanoparticles was imaged by fluorescence and X-ray computed tomography (CT) with an IVIS Spectrum CT (Perkin Elmer) one day following injection. Three-dimensional reconstruction of fluorescent foci around the kidneys was performed with multiple imaging fields and overlaid onto a computed tomographic image of the mouse in order to confirm kidney localization and determine particle distribution throughout the organ.

Fixed organs were dehydrated and paraffin embedded before 5 µm sections were placed on glass slides. The paraffin was removed and the slides were stained with haematoxylin and eosin (H&E) for basic histology. Another set of slides for immunofluorescence were stained with 4',6-diamidino-2-phenylindole (DAPI) to stain nuclei of cells and either an anti-CD31 antibody to stain endothelial cells (Dianova, Cat # DIA-310) with concentration of 1µg/ml or anti-E-cadherin (BD Bioscience; San Jose, CA; Cat# 610181) with concentration of 5 µg/ml to stain epithelial cells. For detection, isotype-specific secondary antibodies conjugated to AlexaFluor 488 (Invitrogen; Carlsbad, CA; Cat# T20922) were used at 1:1000 dilution according to manufacturer's instruction. Immunohistochemistry was performed using a Discovery XT processor (Ventana Medical Systems; Tuscon, AZ). The tissue sections were deparaffinized with EZPrep buffer (Ventana Medical Systems), antigen retrieval was performed with CC1 buffer (Ventana Medical Systems) and sections were blocked for 30 minutes with 10% normal rabbit serum in PBS + 0.1%BSA. Anti-PEG (Abcam; Cambridge, MA; Cat# ab94764, 5ug/ml) antibodies specific to the PEG backbone were applied and sections were incubated for 5 hours, followed by a 60 minute incubation with biotinylated rabbit anti-rat IgG (Vector Labs; Burlingame, CA; Cat#PK-4004) at 1:200 dilution. The assay was performed with a DAB detection kit (Ventana Medical Systems) according to manufacturer instructions. Slides were counterstained with hematoxylin (Ventana Medical Systems) and coverslipped with Permount (Fisher Scientific; Hampton, New Hampshire).

Slides were imaged with an Olympus IX51 inverted light microscope with slide adapter (Olympus; Center Valley, PA) outfitted with an Olympus DP73 digital color camera and Olympus XM10 monochrome camera. Fluorescent slides were excited with filtered light from a X-Cite 120Q lamp (Lumen Dynamics; Ontario, Canada). Fluorescence images were acquired with appropriate filter cubes for DAPI, AlexaFluor 488, and Cy5 with constant exposure times for each fluorophore and analyzed in ImageJ (National Institutes of Health; Bethesda, MD) with constant brightness values for each.

### Nanoparticle Toxicity Study

Mice treated with A- or C-MNPs, dye alone, or PBS alone were weighed immediately preceding injection, on the third day following treatment, and on the seventh day following treatment. The kidneys from mice in each group were sectioned as described herein. Sections were reviewed by a licensed anatomic pathologist for histomorphological evidence of damage.

FIG. 18A shows *ex vivo* organ fluorescence from mice injected with 25 mg/kg A-MNPs or PBS normalized by total organ weight (Mean ± SD), as opposed to 50 mg/kg shown in FIG. 5-13. Data shows up to 25-fold greater localization to the kidneys than any other organ. FIG. 18B shows 25 mg/kg A-MNP injection versus 50 mg/kg. 25 mg/kg A-MNP injected reduced kidney targeting was by half, but almost complete reduction in non-specific targeting to other organs compared to 50 mg/kg MNP injection. FIG. 18C shows specificity of A-MNPs to the kidneys compared to other organs comparing the two doses.

FIG. 19A shows biodistribution of 25 mg/kg A-MNPs over the course of 28 days. Results show specific renal accumulation persists for at least one month, and reaches its maximum at 7 days post-injection. FIG. 19B shows specificity of 25 mg/kg A-MNPs to the kidney compared to other organs increases over time as non-specific accumulation is washed out.

FIG. 20 shows fluorescent dye in the urine of mice injected with A-MNPs, PBS, or dye alone for 48 hours post-injection.

FIGS. 21A - 21D show renal health in mice injected with A-MNPs. FIGS. 21A and 21B show serum nitrogen (FIG. 21A) and creatinine (FIG. 21B) of mice up to 7 days following nanoparticle administration. FIG. 21C shows normalized total protein in the urine in mice treated with A-MNPs for up to 7 days following injection. FIG. 21D shows representative H&E staining of mouse renal sections in mice treated with A-MNPs or PBS alone. These data suggest that selective localization of A-MNPs to kidneys does not affect renal function or cause any apparent toxicity.

FIGS. 22A - 22E show that 25 mg/kg A-MNP injection shows no adverse effects on blood cells in mice.

### Long-Term In vivo Biodistribution Study

Mice in each group were imaged using an IVIS Spectrum Pre-Clinical *In vivo* Imaging System for 7 days as described in the main Materials and Methods. Separately, 3 mice, comprising a mouse treated with PBS alone, A-MNPs, or C-MNPs were imaged every 1-2 weeks for approximately 3 months following injection to measure long-term fluorescence attenuation, which is interpreted to signify MNP degradation.

### PLGA-methoxy PEG Nanoparticle Characterization

Neutral PLGA-mPEG mesoscale nanoparticles (N-MNPs) were synthesized and characterized essentially identically to PLGA nanoparticles functionalized with NH₂-PEG-COOH, which was described in the main Materials and Methods. Instead of NH₂-PEG-COOH, however, NH₂-PEG-methoxy (MW 5kDa) (Nanocs; New York, NY) was conjugated to carboxylic acid-terminated PLGA. The polymer was characterized by 1H NMR. Nanoparticles encapsulating DEDC were formed with this polymer and characterized for size, ζ potential, and encapsulation as described in the main manuscript.

A group of 3 SKH-1 Elite hairless mice were fed with 5V75 chow for at least 1 week before the experiment to reduce fluorescent background from alfalfa-containing food. Mice were injected intravenously via the tail vein with 50 mg/kg of N-MNPs encapsulating DEDC for fluorescence localization studies. One control mouse was injected with 125 µL PBS. These mice were imaged 30 minutes, 3 hours, and 1-3 days following injection with an IVIS Spectrum using 640 nm excitation and 680 nm emission filters. Total combined fluorescence efficiency from both kidneys was measured. At day 3, the mice were euthanized and the following organs were recovered, imaged, and weighed: kidneys, heart, lungs, spleen, and liver.

### PLGA Nanoparticle Characterization

Opsonizing PLGA mesoscale nanoparticles (O-MNPs) were synthesized similarly to PLGA-PEG and PLGA-mPEG nanoparticles as described in the main Materials and Methods. Nanoparticles were formed by nanoprecipiation with PLGA and IR-783 fluorescent dye (25 mg used). The size, ζ potential, and encapsulation efficiency were measured. It should be noted that IR-783 was used instead of DEDC due to precipitate formation with the latter.

A group of 8 SKH-1 Elite hairless mice fed with 5V75 food were injected intravenously via the tail vein with 50 mg/kg of O-MNPs encapsulating IR-783 for fluorescence localization studies. Two control mice were injected with 125 µL PBS. These mice were imaged 30 minutes, 4 hours, and 24 hours following injection with the 647 nm excitation and 820 nm emission filters of an IVIS Spectrum. Four treated mice and one control mouse were euthanized after 24 hours. The remaining mice were imaged at 2 and 3 days following injection before euthanization. Following euthanization, the kidneys, heart, lungs, spleen, and liver were recovered, imaged, and weighed. Total fluorescence efficiency normalized by weight of each organ was obtained.

### Ex vivo to In vivo Comparison

A SKH-1 Elite hairless mouse injected with 50 mg/kg A-MNPs was imaged via an IVIS Spectrum using the 650 nm excitation and 680 nm emission filters 2 days following injection. The mouse was euthanized and kidneys, heart, lungs, spleen, and liver were extracted, leaving other organs intact and in place. The mouse without organs was then imaged as well as the organs alone.

To determine the difference in fluorescence from kidneys *ex vivo* versus *in vivo,* regions of interest (ROIs) were drawn around the kidneys and central lung region and TFE was obtained for each ROI. After euthanization and organ imaging, TFE was obtained for both kidneys and lungs. TFE for kidneys *ex vivo* was divided by that of kidneys *in vivo* to obtain the *in vivo* underestimation ratio. The same method was performed for the lungs.

The difference in nanoparticle fluorescence emission intensity between organs *in vivo* and *ex vivo* was assessed. One mouse, treated with 50 mg/kg A-MNPs, was euthanized 2 days following injection. *In vivo,* prior to euthanization, the fluorescence localization pattern was identical to that shown in similar experiments with the same treatment as shown in FIG. 3A and FIG. 6B. Following euthanization and organ extraction, the carcass lacked the bright foci, confirming that the fluorescence emanated from the removed kidneys as shown in FIG. 9A. Furthermore, the fluorescence in the kidneys *ex vivo* was brighter than all other organs, as previously seen in FIG. 3B with these nanoparticles. Additionally, the *ex vivo* signal from each kidney was 25-30 times higher than the signal *in vivo,* but this phenomenon was not seen in the lungs, which was only 2.0-3.5 times higher *ex vivo* than *in vivo* as shown in FIG. 9B.

### In vitro Nanoparticle Uptake

The following cell lines were used to determine uptake of anionic and cationic nanoparticles *in vitro*: MCF-7 human breast adenocarcinoma (ATCC; Manassas, VA), SK-RC-48 human clear cell renal cell carcinoma (ccRCC) (Weill Cornell Medical College; New York, NY), bEND3 mouse brain endothelial (ATCC), endothelial cell line EA-926, and human kidney proximal tubular epithelial cell line HK-2. MCF-7 cells were cultured in Dulbecco's Modified Eagle Medium (DMEM) with penicillin (10,000 U/mL)/streptomycin (10,000 U/mL)/glutamine (29.2 mg/mL) (Gibco; Carlsbad, CA), 0.01 mg/mL human recombinant insulin (Gibco), and 10% fetal bovine serum (FBS) (Gibco); SK-RC-48 were cultured in DMEM with 1X penicillin/streptomycin/glutamine and 10% FBS; and bEND3 cells in the same plus 1% glutaMAX (Gibco). HK-2 cells were cultured in Keratinocyte Serum Free Media (Gibco). EA-926 cells were cultured in RPMI-1640 medium (Gibco) supplemented with 10% FBS. Cells were passaged approximately weekly and media changed every 2-3 days. Cells were seeded into a 6-well cell culture dish for uptake studies. A-MNPs and C-MNPs in PBS were diluted in the appropriate medium at a final concentration of 100 µg/mL and incubated with each cell line for 10 minutes. Cells were washed with 2 x 1mL PBS before adding fresh media. Fluorescent images were captured with the microscope and camera setup described in the main Materials and Methods section with Cy5 filters 10 minutes following washing. Images were obtained with identical exposure times and processed in ImageJ with identical brightness settings.

FIGS. 15A and 15B show nanoparticle uptake. FIG. 15A shows that MNPs are taken up into the endothelial cell line EA-926 and the human kidney proximal tubular epithelial cell line HK-2. FIG. 15B shows that nanoparticle uptake into HK-2 cells can be blocked by incubation at 4C, indicating an energy-dependent uptake mechanism.

FIG. 16 shows nanoparticle uptake in human proximal tubular epithelial HK-2 cells. Cells were incubated with 100 µg/mL NPs in 1 mL of their respective complete medias for 30 minutes. Cells were washed 3x with PBS and fresh media was added. For energy-dependent uptake analysis, cells were incubated at 4°C for 30 minutes prior to nanoparticle addition and during incubation. Cells were imaged with a fluorescence microscope using Cy5 filters.

FIG. 17 shows MNP uptake into HK-2 cells can be inhibited by Dynasore, an inhibitor of clathrin/caveolin-mediated endocytosis.

### Exemplary Disease Targets

Table 3 shows exemplary disease targets, therapeutics (e.g., classes of drugs/inhibitors), and a brief rationale. In certain embodiments, therapeutics can be encapsulated within the MNPs.

Because of the kidney-targeting ability of the drug carrier nanoparticles, treatment of the kidney can now be performed using therapeutic agents that are currently not used for kidney treatment and/or that are currently experimental. Various therapeutic agents (e.g., TGFb inhibitors, mTOR inhibitors, everolimus, kinase inhibitors) have not been usable for kidney disease treatment due to poor pharmacokinetics. By the time the kidneys respond to a delivered therapeutic agent, toxicity side-effects occur elsewhere in the body. Accordingly, the mesoscale nanoparticles disclosed herein can selectively deliver therapeutic agents (e.g., therapeutic agents that are typically not used for kidney disease treatment due to poor pharmacokinetics) to the kidney while minimizing toxicity elsewhere in the body.

**Table 3**

| Disease | Target | Therapeutic | Rationale |
|---|---|---|---|
| Acute Kidney Injury | TGFbeta Receptor | Kinase inhibitors- | The proximal tubule epithelial cells express high levels of TGFβ and its receptors. TGFβ promotes proximal tubule cell apoptosis and thus plays a role in multiple forms of AKI. TGFβ signaling also retards recovery of tubular epithelial cells by inhibition of their proliferation and re-differentiation. |
| | | e.g. LY2157299 (galunisertib) (4-[2-(6-methylpyridin-2-yl)-5,6-dihydro-4H-pyrrolo[1,2-b]pyrazol-3-yl]quinoline-6-carboxamide), SD-208 (2-(5-Chloro-2-fluorophenyl)-4-[(4-pyridyl)amino]pteridine), SB505124 (2-[4-(1,3-benzodioxol-5-yl)-2-tert-butyl-1H-imidazol-5-yl]-6-methylpyridine) | |
| | | | Conditional TGFβRII knock-out mice lacking TGFβRII have milder forms of AKI and are able to recover more quickly. |
| Acute Kidney Injury | Reactive Oxygen Species and DNA Damage | ROS inhibitors- | Amifostine has been shown to ameliorate cisplatin-induced acute kidney injury in pre-clinical and clinical trials. |
| | | e.g. Amifostine (2-(3-aminopropylamino)ethylsulfanyl phosphonic acid) | |
| Acute Kidney Injury | Nf kappa B | Inhibitors- | Increased activity of NF-κB plays a major role in the pathogenesis of AKI and an increase in pro-inflammatory cytokines levels mediated in part by NF-kB activation predicts mortality in AKI. Inhibition of NF-κB transcription attenuated cisplatin-induced acute kidney injury in mice. |
| | | e.g. Pyrrolidine dithiocarbamate (Pyrrolidine-1-carbodithioic acid), quinazoline (Quinazoline), BMS-345541 (4-(2'-Aminoethyl)amino-1,8-dimethylimidazo[1,2-a]quinoxaline), BAY-11-7085 ((2E)-3-[[4-(1,1-Dimethylethyl)phenyl]sulfonyl]-2-propenenitrile) | |
| Chronic Kidney Disease | p21 | siRNA or small-molecule inhibitors | In CKD, p21-dependent cell cycle arrest reduced renal growth and repair, causing a more hypertrophic response and leading to further damage due to increased work load. |
| Chronic Kidney Disease | TGFbeta Receptor | Kinase inhibitors- | Tubular epithelial cell apoptosis in CKD models is reduced by TGFβ inhibition, TGFβ is a strong inducer of epithelial-mesenchymal transition (EMT) in tubular cells, and an increase in extracellular matrix (ECM) and inflammatory cytokine production by tubular cells is due to TGFβ. |
| | | e.g. LY2157299 (galunisertib) (4-[2-(6-methylpyridin-2-yl)-5,6-dihydro-4H-pyrrolo[1,2-b]pyrazol-3-yl]quinoline-6-carboxamide), SD-208 (2-(5-Chloro-2-fluorophenyl)-4-[(4-pyridyl)amino]pteridine), SB505124 (2-[4-(1,3-benzodioxol-5-yl)-2-tert-butyl-1H-imidazol-5-yl]-6-methylpyridine) | |
| Polycystic Kidney Disease (PKD) | mTOR | Inhibitors-e.g. | Activation of mTOR is linked to tubular cell proliferation in both animal models and human PKD. Inhibitors of mTOR may attenuate cyst growth and interstitial fibrosis by inhibiting vascular remodeling, angiogenesis, and fibrogenesis. |
| | | RAD001 (everolimus)( dihydroxy-12-[(2R)-1-[(1S,3R,4R)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0 hexatriaconta-16,24,26,28-tetraene-2,3,10,14,20-pentone) | |
| Clear Cell Renal Cell Carcinoma (ccRCC) | Glutaminase | Inhibitors-e.g. BPTES (N,N'-[Sulfanediylbis(2,1-ethanediyl-1,3,4-thiadiazole-5,2-diyl)]bis(2-phenylacetamide) ), CB-839 (2-(pyridin-2-yl)-N-(5-(4-(6-(2-(3-(trifluoromethoxy)phenyl)acetamido)pyridazin-3-yl)butyl)-1,3,4-thiadiazol-2-yl)acetamide) | Glutaminase is overexpressed in human ccRCC. Glutaminase promotes aberrant energetic processes in ccRCC. |

## Claims

1. A mesoscale nanoparticle composition comprising:
a core comprising poly(lactic-co-glycolic acid) (PLGA); and
a surface coating comprising polyethylene glycol having a surface charge between -40 mV to +40 mV, and
wherein the composition further comprises at least one of:
i) a therapeutic agent wherein the therapeutic agent comprises a targeted chemotherapeutic, a metabolic targeting therapeutic, an alpha-7 nicotine receptor antagonist, a hypertension therapeutic; a TGFbeta inhibitor, a reactive oxygen species and DNA damage inhibitor, a Nf kappa B inhibitor, a p21 inhibitor; a mTOR inhibitor, or a glutaminase inhibitor; and
ii) a positron emission tomography (PET) tracer, a dye, or a fluorescent molecule within the core, and
wherein the composition is in the form of a nanoparticle having an average diameter from 250 nm to 500 as determined by dynamic light scattering in aqueous solution.

2. The mesoscale nanoparticle composition of claim 1, wherein a base of PLGA is modified.

3. The mesoscale nanoparticle composition of claim 2, wherein the PLGA has a molecular weight from 7 kDa to 54 kDa.

4. The mesoscale nanoparticle composition of any one of claims 1-3, wherein the polyethylene glycol has a molecular weight from 2 kDa to 10 kDa.

5. The mesoscale nanoparticle composition of any one of claims 1-4, wherein the surface coating is selected from the group consisting of polyethylene glycol (PEG), PEGcarboxylic acid, PEG-carboxylic acid-DMAB, and methoxy PEG.

6. The mesoscale nanoparticle composition of claim 6, wherein the weight ratio of PEG to PLGA is from 9% to 13% prior to synthesis of the mesoscale nanoparticle composition.

7. The mesoscale nanoparticle composition of any one of claims 1-6, wherein the targeted chemotherapeutic is doxorubicin, sorafenib, or sunitinib; and/or
the metabolic targeting therapeutic is STF-31, CPI- 613, or Fasentin; and/or the TGFbeta inhibitor is LY2157299, SD-208, or SB505124; and/or the Nf kappa B inhibitor is Pyrrolidine dithiocarbamate, quinazoline, BMS-345541, or BAY-11-7085.

8. The mesoscale nanoparticle composition of claim 1, wherein the therapeutic agent is noncovalently associated with the nanoparticle, optionally where the therapeutic agent is encapsulated within the surface coating.

9. The mesoscale nanoparticle composition of any one of claims 1-8, wherein a radiolabel is noncovalently associated with the nanoparticle and/or attached to the nanoparticle.

10. A mesoscale nanoparticle composition as defined in any one of claims 1-9, for use in treating a patient suffering from or susceptible to a disease or condition affecting the kidney, wherein the disease or condition is a member selected from the group consisting of renal carcinoma, acute kidney disease, chronic kidney disease, polycystic kidney disease, heart failure, liver cirrhosis, hypertension, and renal failure.

11. A method for monitoring a patient, the method comprising administering the mesoscale nanoparticle composition of any one of claims 1-9 to the patient for investigating renal blood flow or renal physiology, wherein the mesoscale nanoparticle composition comprises an imaging agent; and imaging the administered mesoscale nanoparticle composition.

12. The method of claim 11, wherein the administered mesoscale nanoparticle composition demonstrates selective targeting of kidneys of the patient such that concentration of the mesoscale nanoparticle composition in the kidneys is at least 1.5 times greater than concentration of the mesoscale nanoparticle composition in any of the heart, lung, liver, or spleen of the patient at a given time following administration of the composition, wherein the given time is from 3 days to 2 months following administration.

## Patentansprüche

1. Mesoskalige Nanopartikelzusammensetzung, umfassend:
einen Kern, umfassend Poly(milch-co-glykolsäure) (PLGA); und
Oberflächenbeschichtung, umfassend Polyethylenglykol, das eine Oberflächenladung zwischen - 40 mV und +40 mV aufweist, und
wobei die Zusammensetzung ferner mindestens eines von Folgendem umfasst:
i) ein therapeutisches Mittel, wobei das therapeutische Mittel ein zielgerichtetes Chemotherapeutikum, ein auf den Stoffwechsel abzielendes Therapeutikum, einen Alpha-7-Nikotinrezeptor-Antagonisten, ein Hypertonie-Therapeutikum, einen TGFbeta-Inhibitor, einen Inhibitor für reaktive Sauerstoffspezies und DNA-Schäden, einen Nf kappa B-Inhibitor, einen p21-Inhibitor, einen mTOR-Inhibitor oder einen Glutaminase-Inhibitor umfasst; und
ii) einen Positronen-Emissions-Tomographie (PET)-Tracer, einen Farbstoff oder ein fluoreszierendes Molekül im Inneren des Kerns und
wobei die Zusammensetzung die Form eines Nanopartikels mit einem durchschnittlichen Durchmesser von 250 nm bis 500 aufweist, wie durch dynamische Lichtstreuung in wässriger Lösung bestimmt.

2. Mesoskalige Nanopartikelzusammensetzung nach Anspruch 1, wobei eine Basis aus PLGA modifiziert ist.

3. Mesoskalige Nanopartikelzusammensetzung nach Anspruch 2, wobei das PLGA ein Molekulargewicht von 7 kDa bis 54 kDa aufweist.

4. Mesoskalige Nanopartikelzusammensetzung nach einem der Ansprüche 1 bis 3, wobei das Polyethylenglykol ein Molekulargewicht von 2 kDa bis 10 kDa aufweist.

5. Mesoskalige Nanopartikelzusammensetzung nach einem der Ansprüche 1 bis 4, wobei die Oberflächenbeschichtung ausgewählt ist aus der Gruppe, bestehend aus Polyethylenglykol (PEG), PEG-Carbonsäure, PEG-Carbonsäure-DMAB und Methoxy-PEG.

6. Mesoskalige Nanopartikelzusammensetzung nach Anspruch 6, wobei das Gewichtsverhältnis von PEG zu PLGA vor der Synthese der mesoskaligen Nanopartikelzusammensetzung 9% bis 13% beträgt.

7. Mesoskalige Nanopartikelzusammensetzung nach einem der Ansprüche 1 bis 6, wobei das zielgerichtete Chemotherapeutikum Doxorubicin, Sorafenib oder Sunitinib ist; und/oder
das auf den Stoffwechsel zielende Therapeutikum STF-31, CPI-613 oder Fasentin ist; und/oder
der TGFbeta-Inhibitor LY2157299, SD-208 oder SB505124 ist; und/oder
der Nf kappa B-Inhibitor Pyrrolidindithiocarbamat, Chinazolin, BMS-345541 oder BAY-11-7085 ist.

8. Mesoskalige Nanopartikelzusammensetzung nach Anspruch 1, wobei der therapeutische Wirkstoff nicht kovalent mit dem Nanopartikel assoziiert ist, wobei der therapeutische Wirkstoff optional in der Oberflächenbeschichtung eingekapselt ist.

9. Mesoskalige Nanopartikelzusammensetzung nach einem der Ansprüche 1 bis 8, wobei ein radioaktiver Marker nicht-kovalent mit dem Nanopartikel assoziiert und/oder an den Nanopartikel gebunden ist.

10. Mesoskalige Nanopartikelzusammensetzung, wie in einem der Ansprüche 1 bis 9 definiert, zur Verwendung bei der Behandlung eines Patienten, der an einer Krankheit oder einem Zustand leidet oder dafür empfänglich ist, die bzw. der die Niere betrifft, wobei die Krankheit oder der Zustand ein Element ist, das aus der Gruppe ausgewählt ist, die aus Nierenkarzinom, akuter Nierenerkrankung, chronischer Nierenerkrankung, polyzystischer Nierenerkrankung, Herzversagen, Leberzirrhose, Bluthochdruck und Nierenversagen besteht.

11. Verfahren zur Überwachung eines Patienten, wobei das Verfahren die Verabreichung der mesoskaligen Nanopartikelzusammensetzung nach einem der Ansprüche 1 bis 9 an den Patienten zur Untersuchung des Nierendurchblutungsflusses oder der Nierenphysiologie umfasst, wobei die mesoskalige Nanopartikelzusammensetzung ein Abbildungsmittel umfasst; und die Abbildung der verabreichten mesoskaligen Nanopartikelzusammensetzung.

12. Verfahren nach Anspruch 11, wobei die verabreichte mesoskalige Nanopartikelzusammensetzung eine selektive Ausrichtung auf die Nieren des Patienten zeigt, sodass die Konzentration der mesoskaligen Nanopartikelzusammensetzung in den Nieren mindestens 1,5 mal höher ist als die Konzentration der mesoskaligen Nanopartikelzusammensetzung in Herz, Lunge, Leber oder Milz des Patienten zu einem gegebenen Zeitpunkt nach der Verabreichung der Zusammensetzung, wobei der gegebene Zeitpunkt zwischen 3 Tagen und 2 Monaten nach der Verabreichung liegt.

## Revendications

1. Composition de nanoparticules moyennes comprenant :
un noyau comprenant de l'acide poly(lactique-co-glycolique) (PLGA) ; et
un revêtement de surface comprenant du polyéthylène glycol ayant une charge de surface comprise entre -40 mV et +40 mV, et
dans laquelle la composition comprend en outre au moins l'un des éléments suivants :
i) un agent thérapeutique, dans laquelle l'agent thérapeutique comprend un agent chimiothérapeutique ciblé, un agent thérapeutique de ciblage métabolique, un antagoniste du récepteur alpha-7 de la nicotine, un agent thérapeutique contre l'hypertension ; un inhibiteur du TGF-bêta, un inhibiteur des espèces réactives de l'oxygène et des dommages à l'ADN, un inhibiteur de Nf kappa B, un inhibiteur de p21 ; un inhibiteur de mTOR ou un inhibiteur de la glutaminase ; et
ii) un traceur de tomographie par émission de positons (TEP), un colorant, ou une molécule fluorescente dans le noyau, et
dans laquelle la composition se présente sous la forme d'une nanoparticule ayant un diamètre moyen de 250 nm à 500 tel que déterminé par diffusion dynamique de la lumière en solution aqueuse.

2. Composition de nanoparticules moyennes selon la revendication 1, dans laquelle une base de PLGA est modifiée.

3. Composition de nanoparticules moyennes selon la revendication 2, dans laquelle le PLGA a un poids moléculaire de 7 kDa à 54 kDa.

4. Composition de nanoparticules moyennes selon l'une quelconque des revendications 1 à 3, dans laquelle le polyéthylène glycol a un poids moléculaire de 2 kDa à 10 kDa.

5. Composition de nanoparticules moyennes selon l'une quelconque des revendications 1 à 4, dans laquelle le revêtement de surface est choisi dans le groupe constitué du polyéthylène glycol (PEG), de l'acide PEG-carboxylique, de l'acide PEG-carboxylique-DMAB et du méthoxy PEG.

6. Composition de nanoparticules moyennes selon la revendication 6, dans laquelle le rapport pondéral du PEG au PLGA est de 9% à 13% avant la synthèse de la composition de nanoparticules moyennes.

7. Composition de nanoparticules moyennes selon l'une quelconque des revendications 1 à 6, dans laquelle l'agent chimiothérapeutique ciblé est la doxorubicine, le sorafénib ou le sunitinib ; et/ou
l'agent thérapeutique de ciblage métabolique est le STF-31, le CPI-613 ou le Fasentin ; et/ou
l'inhibiteur du TGFbêta est le LY2157299, le SD-208 ou le SB505124 ; et/ou
l'inhibiteur de Nf kappa B est le dithiocarbamate de pyrrolidine, la quinazoline, le BMS-345541 ou le BAY-11-7085.

8. Composition de nanoparticules moyennes selon la revendication 1, dans laquelle l'agent thérapeutique est associé de manière non covalente à la nanoparticule, éventuellement dans laquelle l'agent thérapeutique est encapsulé à l'intérieur du revêtement de surface.

9. Composition de nanoparticules moyennes selon l'une quelconque des revendications 1 à 8, dans laquelle un radiomarqueur est associé de manière non covalente à la nanoparticule et/ou attaché à la nanoparticule.

10. Composition de nanoparticules moyennes telle que définie dans l'une quelconque des revendications 1 à 9, destinée à être utilisée dans le traitement d'un patient souffrant ou susceptible d'être atteint d'une maladie ou d'une affection affectant le rein, dans laquelle la maladie ou l'affection est un élément choisi dans le groupe constitué du carcinome rénal, d'une maladie rénale aiguë, d'une maladie rénale chronique, d'une maladie rénale polykystique, de l'insuffisance cardiaque, de la cirrhose du foie, de l'hypertension et de l'insuffisance rénale.

11. Procédé de surveillance d'un patient, le procédé comprenant l'administration de la composition de nanoparticules moyennes selon l'une quelconque des revendications 1 à 9 au patient pour étudier le flux sanguin rénal ou la physiologie rénale, dans lequel la composition de nanoparticules moyennes comprend un agent d'imagerie ; et l'imagerie de la composition de nanoparticules moyennes administrée.

12. Procédé selon la revendication 11, dans lequel la composition de nanoparticules moyennes administrée démontre un ciblage sélectif des reins du patient de sorte que la concentration de la composition de nanoparticules moyennes dans les reins est au moins 1,5 fois supérieure à la concentration de la composition de nanoparticules moyennes dans l'un quelconque du cœur, des poumons du le foie ou de la rate du patient à un moment donné après l'administration de la composition, dans lequel le temps donné est de 3 jours à 2 mois après l'administration.
